# EUROPÄISCHE PATENTSCHRIFT

(11) **EP 3 068 773 B1**
(45) Veröffentlichungstag und Bekanntmachung des Hinweises auf die Patenterteilung: **05.07.2017**
(21) Anmeldenummer: 14802608.1
(22) Anmeldetag: 10.11.2014
(51) Int. Cl.: C07D 401/04, C07D 405/14, C07D 409/14, A01N 43/58, A01P 13/00

(54) **PYRIDAZINON-DERIVATE UND IHRE VERWENDUNG ALS HERBIZIDE**
PYRIDAZINONE DERIVATIVES AND THEIR USE AS HERBICIDES
DÉRIVÉS DE PYRIDAZINONE ET LEUR UTILISATION EN TANT QU'HERBICIDES

(30) Priorität: 12.11.2013 EP 13192411
(43) Veröffentlichungstag der Anmeldung: 21.09.2016
(73) Patentinhaber: Bayer CropScience Aktiengesellschaft, 40789 Monheim am Rhein (DE)
(72) Erfinder: BRAUN, Ralf, 76857 Ramberg (DE); WALDRAFF, Christian, 61118 Bad Vilbel (DE); HEINEMANN, Ines, 65719 Hofheim (DE); KÖHN, Arnim, 55270 Klein-Winternheim (DE); AHRENS, Hartmut, 63329 Egelsbach (DE); ANGERMANN, Alfred, 65830 Kriftel (DE); LINDELL, Stephen, David, 65779 Kelkheim (DE); SCHMUTZLER, Dirk, 65795 Hattersheim (DE); DIETRICH, Hansjörg, 65835 Liederbach (DE); ROSINGER, Christopher, Hugh, 65719 Hofheim am Taunus (DE); GATZWEILER, Elmar, 61231 Bad Nauheim (DE)
(74) Vertreter: BIP Patents
(86) Internationale Anmeldenummer: PCT/EP2014/074127
(87) Internationale Veröffentlichungsnummer: WO 2015/071205

(56) Entgegenhaltungen:
- WO-A1-2011/045271
- WO-A1-2013/050421

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO2013/050421 A1 sind Pyridazinone als Herbizide beschrieben. Allerdings zeigen diese Wirkstoffe nicht immer eine ausreichende Wirkung gegen Schadpflanzen und/oder sie sind zum Teil nicht ausreichend verträglich mit einigen wichtigen Kulturpflanzen, wie Getreidearten, Mais und Reis.

Aufgabe der vorliegenden Erfindung ist es, alternative herbizid wirksame Wirkstoffe bereitzustellen. Diese Aufgabe wird durch die nachfolgend beschriebenen erfindungsgemäßen Pyridazinon-derivate gelöst.

Ein Gegenstand der vorliegenden Erfindung sind somit Pyridazinon-derivate der Formel (I) oder deren Salze 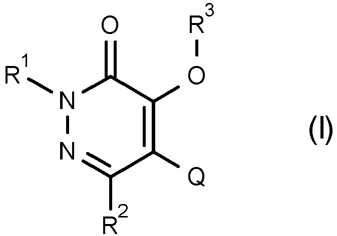 worin
Q bedeutet Q¹ oder Q², 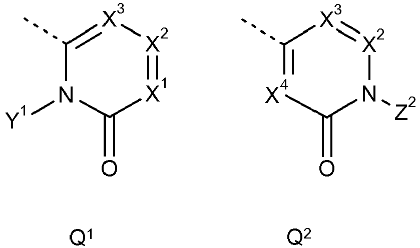
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, (C₃-C₆)-Cycloalkyl, (C₃-C₆)-Cycloalkyl-(C₁-C₆)-alkyl, (C₁-C₆)-Alkoxy-(C₁-C₆)-alkyl, Tetrahydropyran-2-yl oder durch s Reste R⁴ sustituiertes Benzyl,
R² bedeutet Wasserstoff, Halogen, Cyano, Hydroxy, Nitro, Amino,(C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₁-C₆)-alkyl-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy, Halogen-(C₁-C₆)-alkoxy, (C₁-C₆)-Alkyl-S(O)ₙ, Halogen-(C₁-C₆)-alkyl-S(O)ₙ, (C₁-C₆)-Alkyl-S(O)ₙ-(C₁-C₃)-alkyl, Halogen-(C₁-C₆)-alkyl-S(O)ₙ-(C₁-C₃)-alkyl, (C₁-C₆)-Alkylamino oder (C₁-C₆)-Dialkylamino,
R³ bedeutet Wasserstoff, (C₁-C₆)-Alkylcarbonyl, (C₁-C₆)-Alkoxycarbonyl, (C₁-C₆)-Alkyl-S(O)ₙ, (C₁-C₆)-Alkyl-S-carbonyl, Arycarbonyl oder Aryl S(O)ₙ bedeutet, wobei die zwei letztgenannten Reste jeweils durch s Reste R⁴ substituiert sind,
   R⁴ bedeutet Halogen, (C₁-C₄)-Alkyl, Halogen-(C₁-C₄)-alkyl, (C₁-C₄)-Alkoxy,
   X¹ bedeutet N oder CZ¹,
   X² bedeutet N oder CW,
   X³ bedeutet N oder CR⁵,
   X⁴ bedeutet N oder CY²,
   Y¹ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, OCOOR⁶, OC(O)N(R⁶)₂, OR⁶, OCOR⁶, OSO₂R⁷, (C₁-C₆)-Alkyl-S(O)ₙR⁷, (C₁-C₆)-Alkyl-OR⁶, (C₁-C₆)-Alkyl-OCOR⁶, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-CN, (C₁-C₆)-Alkyl-SO₂N(R⁶)₂, (C₁-C₆)-Alkyl-NR⁶COR⁶, (C₁-C₆)-Alkyl-NR⁶SO₂R⁷, CH₂P(O)(OR¹⁰)₂, (C₁-C₆)-Alkyl-Aryl, (C₁-C₆)-Alkyl-Heteroaryl, (C₁-C₆)-Alkyl-Heterocyclyl, wobei die drei letztgenannten Reste jeweils durch s Reste aus der Gruppe bestehend aus Halogen, Cyano, Nitro, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
   Y² bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-Alkyl-S(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die sechs letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
   Z¹ bedeutet Wasserstoff, Halogen, Cyano, Rhodano, Nitro, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₁-C₆)-alkyl-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)2, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, P(O)(OR¹⁰)₂, Heteroaryl, Heterocyclyl oder Phenyl, wobei die drei letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl n Oxogruppen trägt,
   Z² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₁-C₆)-alkyl-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, Heteroaryl, Heterocyclyl oder Phenyl, wobei die drei letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
   W bedeutet Wasserstoff, Halogen, Cyano, Nitro, (C₁-C₄)-Alkyl, (C₁-C₄)-Haloalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, (C₁-C₄)-Alkoxy oder (C₁-C₄)-Haloalkoxy, oder
   Z¹ oder Z² und W bilden gemeinsam mit den beiden Kohlenstoffatomen, an denen sie gebunden sind, einen fünf- oder sechsgliedrigen Ring bestehend aus t Kohlenstoffatomen und p Heteroatomen aus der Gruppe bestehend aus N, O und S, wobei dieser Ring durch q Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₆)-alkyl substituiert ist,
   R⁵ bedeutet Wasserstoff, Halogen, (C₁-C₄)-Alkyl, Halogen-(C₁-C₄)-alkyl oder (C₁-C₄)-Alkoxy,
   R⁶ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR⁸-Heteroaryl, (C₁-C₆)-Alkyl-NR⁸-Heterocyclyl wobei die 21 letztgenannten Reste durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO2R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
   R⁷ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl, wobei diese 17 Reste durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
   R⁸ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
   R⁹ bedeutet (C₁-C₆)-Alkyl, (C₂-C₆)-Alkenyl, (C₂-C₆)-Alkinyl oder Phenyl,
   R¹⁰ bedeutet (C₁-C₄)-Alkyl,
   n bedeutet 0, 1 oder 2,
   p bedeutet 0, 1, 2 oder 3,
   q bedeutet 0, 1, 2, 3 oder 4,
   s bedeutet 0, 1, 2, 3, 4 oder 5,
   t bedeutet 0, 1, 2, 3, 4, 5 oder 6.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.
Heterocyclyl bedeutet einen gesättigten, teilgesättigten oder vollständig ungesättigten cyclischen Rest, der 3 bis 6 Ringatome enthält, von denen 1 bis 4 aus der Gruppe bestehend aus Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe bestehend aus Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1 H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.
Die Verbindungen der Formel (I) können Salze bilden. Salzbildung kann durch Einwirkung einer Base auf Verbindungen der Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate
und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NR^{a}R^{b}R^{c}R^{d}]⁺, worin R^{a} bis R^{d} jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Bevorzugt sind Verbindungen der allgemeinen Formel (I), worin
Q bedeutet Q¹ oder Q²,
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, (C₃-C₆)-Cycloalkyl, (C₃-C₆)-Cycloalkyl-(C₁-C₆)-alkyl oder (C₁-C₆)-Alkoxy-(C₁-C₆)-alkyl,
R² bedeutet Wasserstoff, Halogen, Cyano, (C₁-C₆)-Alkyl, Halogen-(C₁-C₆)-alkyl, (C₂-C₆)-Alkenyl, (C₂-C₆)-Alkinyl oder(C₁-C₆)-Alkyl-S(O)ₙ,
R³ bedeutet Wasserstoff,
R⁴ bedeutet Halogen, (C₁-C₄)-Alkyl, Halogen-(C₁-C₄)-alkyl, (C₁-C₄)-Alkoxy,
X¹ bedeutet N oder CZ¹,
X² bedeutet N oder CW,
X³ bedeutet N oder CH,
X⁴ bedeutet N oder CY²,
Y¹ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, OCOOR⁶, OC(O)N(R⁶)₂, OR⁶, OCOR⁶, OSO₂R⁷, (C₁-C₆)-Alkyl-S(O)ₙR⁷, (C₁-C₆)-Alkyl-OR⁶, (C₁-C₆)-Alkyl-OCOR⁶, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR⁶SO₂R⁷, CH₂P(O)(OR¹⁰)₂, (C₁-C₆)-Alkyl-Aryl, (C₁-C₆)-Alkyl-Heteroaryl, (C₁-C₆)-Alkyl-Heterocyclyl, wobei die drei letztgenannten Reste jeweils durch s Reste aus der Gruppe bestehend aus Halogen, Cyano, Nitro, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y² bedeutet Wasserstoff, Halogen, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, OCOOR⁶, OC(O)N(R⁶)₂, OR⁶, OCOR⁶, OSO₂R⁷, (C₁-C₆)-Alkyl-S(O)ₙR⁷, (C₁-C₆)-Alkyl-OR⁶, (C₁-C₆)-Alkyl-OCOR⁶, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR⁶SO₂R⁷, CH₂P(O)(OR¹⁰)₂, (C₁-C₆)-Alkyl-Phenyl, (C₁-C₆)-Alkyl-Heteroaryl, (C₁-C₆)-Alkyl-Heterocyclyl, wobei die drei letztgenannten Reste jeweils durch s Reste aus der Gruppe bestehend aus Halogen, Cyano, Nitro, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Z¹ bedeutet Wasserstoff, Halogen, Cyano, Rhodano, Nitro, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, P(O)(OR¹⁰)₂, Heteroaryl, Heterocyclyl oder Phenyl, wobei die letzten drei Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl n Oxogruppen trägt,
Z² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, Heteroaryl, Heterocyclyl oder Phenyl, wobei die letzten drei Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
W bedeutet Wasserstoff, Halogen, (C₁-C₄)-Alkyl, (C₁-C₄)-Haloalkyl, (C₂-C₆)-Alkenyl, (C₂-C₆)-Alkinyl, (C₃-C₆)-Cycloalkyl, (C₁-C₆)-Alkyl-O-(C₁-C₆)-alkyl, (C₃-C₆)-Cycloalkyl-(C₁-C₆)-alkyl, (C₁-C₄)-Alkoxy oder (C₁-C₄)-Haloalkoxy,
R⁶ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR⁸-Heteroaryl, (C₁-C₆)-Alkyl-NR⁸-Heterocyclyl wobei die 21 letztgenannten Reste durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO2R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
R⁷ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl, wobei diese 17 Reste durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
R⁸ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁹ bedeutet (C₁-C₆)-Alkyl, (C₂-C₆)-Alkenyl, (C₂-C₆)-Alkinyl oder Phenyl,
R¹⁰ bedeutet (C₁-C₄)-Alkyl,
n bedeutet 0, 1 oder 2,
p bedeutet 0, 1, 2 oder 3,
q bedeutet 0, 1, 2, 3 oder 4,
s bedeutet 0, 1, 2, 3, 4 oder 5,
t bedeutet 0, 1, 2, 3, 4, 5 oder 6.

Besonders bevorzugt sind Verbindungen der allgemeinen Formel (I), worin
Q bedeutet Q¹ oder Q²,
R¹ bedeutet (C₁-C₄)-Alkyl, Halogen-(C₁-C₃)-alkyl, (C₂-C₄)-Alkenyl, Propargyl, Cyclopropyl oder Cyclopropylmethyl,
R² bedeutet Wasserstoff, Halogen oder (C₁-C₆)-Alkyl,
R³ bedeutet Wasserstoff,
X¹ bedeutet N oder CZ¹,
X² bedeutet N oder CW,
X³ bedeutet N oder CH,
X⁴ bedeutet N oder CY²,
Y¹ bedeutet (C₁-C₆)-Alkyl, Halogen-(C₁-C₆)-alkyl, (C₃-C₆)-Cycloalkyl, OR⁶, S(O)ₙR⁷, (C₁-C₆)-Alkyl-S(O)ₙR⁷, (C₁-C₆)-Alkyl-OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR⁶SO₂R⁷, (C₁-C₆)-Alkyl-Heteroaryl, (C₁-C₆)-Alkyl-Heterocyclyl, wobei die beiden letztgenannten Reste jeweils durch s Reste aus der Gruppe bestehend aus Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y² bedeutet (C₁-C₆)-Alkyl, Halogen-(C₁-C₆)-alkyl, (C₃-C₆)-Cycloalkyl, OR⁶, S(O)ₙR⁷, (C₁-C₆)-Alkyl-S(O)ₙR⁷, (C₁-C₆)-Alkyl-OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR⁶SO₂R⁷, (C₁-C₆)-Alkyl-Heteroaryl, (C₁-C₆)-Alkyl-Heterocyclyl, wobei die beiden letztgenannten Reste jeweils durch s Reste aus der Gruppe bestehend aus Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Z¹ bedeutet Wasserstoff, Halogen, Cyano, Rhodano, Nitro, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, P(O)(OR¹⁰)₂, Heteroaryl, Heterocyclyl oder Phenyl, wobei die 3 letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Z² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, P(O)(OR¹⁰)₂, Heteroaryl, Heterocyclyl oder Phenyl, wobei die 3 letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
W bedeutet Wasserstoff, Halogen, (C₁-C₄)-Alkyl,
R⁶ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR⁸-Heteroaryl, (C₁-C₆)-Alkyl-NR⁸-Heterocyclyl wobei die 21 letztgenannten Reste jeweils durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO2R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
R⁷ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl, wobei diese 17 Reste jeweils durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
R⁸ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁹ bedeutet (C₁-C₆)-Alkyl, (C₂-C₆)-Alkenyl, (C₂-C₆)-Alkinyl oder Phenyl,
R¹⁰ bedeutet (C₁-C₄)-Alkyl,
n bedeutet 0, 1 oder 2,
p bedeutet 0, 1, 2 oder 3,
q bedeutet 0, 1, 2, 3 oder 4,
s bedeutet 0, 1, 2, 3, 4 oder 5,
t bedeutet 0, 1, 2, 3, 4, 5 oder 6.

In allen nachfolgend genannten Formeln haben die Substituenten und Symbole, sofern nicht anders definiert, dieselbe Bedeutung wie unter Formel (I) beschrieben.

Erfindungsgemäße Verbindungen können beispielsweise gemäß Schema 1 analog den in WO2013/050421 A1 genannten Methoden hergestellt werden. 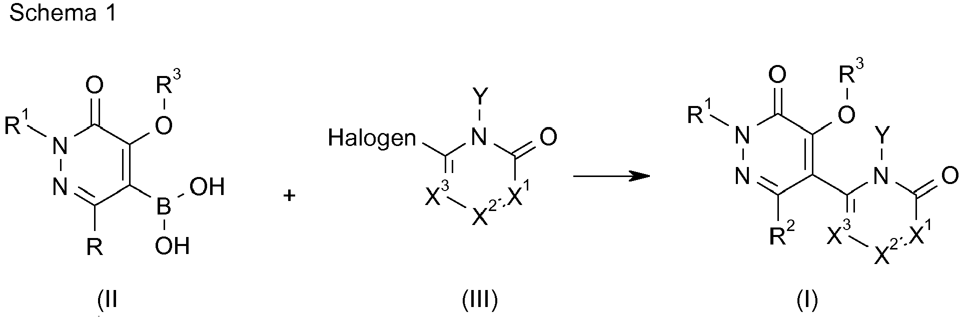 Die Azin-2-on-6-halogenide (III) können z.B. nach Schema 2 hergestellt werden: Die Azin-2-on-6-halogenide (III) können z.B. nach Schema 2 hergestellt werden:

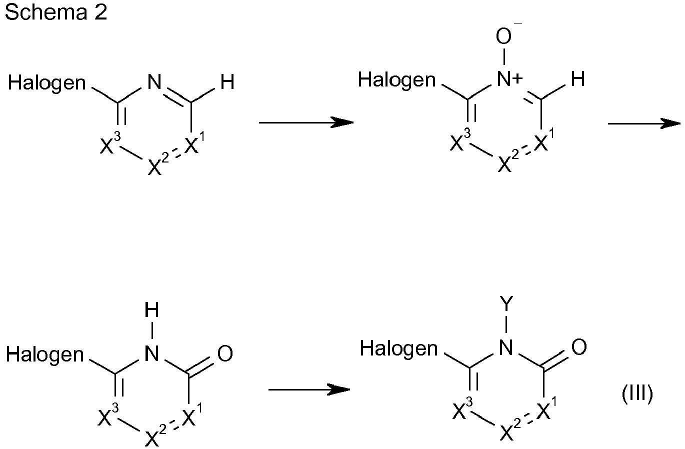

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.
Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, Iowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfasst.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im Einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria und Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola und Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüber hinaus weisen die erfindungsgemäßen Verbindungen, abhängig von ihrer jeweiligen chemischen Struktur und der ausgebrachten Aufwandmenge, hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Des Weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Maniok, Tomate, Erbse und anderen Gemüsesorten.

Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl-oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie
Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.
Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### 1. Synthese von 4-Hydroxy-2-methyl-5-(1-methyl-3-(trifluormethyl)pyrid-2-on-6-yl) pyridazin-3-on (Tabellenbeispiel Nr. 1-4)

Eine Mischung aus 1.75 g (10 mmol) 5-Chlor-4-methoxy-2-methyl-pyridazin-3-on, 5.08 g (20 mmol) Bis(pinacolato)diboran, 0.45 g (1.6 mmol) Tricyclohexylphosphin, 1.47 g (15 mmol) Kaliumacetat und 0.37 g (0.4 mmol) Tris(dibenzylidenaceton)dipalladium unter Argen in 50 ml Dioxan 30 min unter Rückfluß erhitzt. Nach Abkühlen auf 40°C werden 4.52 g (20 mmol) 2-Brom-5-trifluormethyl-pyridin, 6.08 g (40 mmol) Cäsiumfluorid und 0,59 g (.08 mmol) PdCl₂dppf zugegeben und anschließend wieder 3.5 h unter Rückfluß erhitzt. Nach Abkühlen wird über Kieselgur abgesaugt, mit Essigester gewaschen, das Filtrat eingeengt und der Rückstand direkt säulenchromatographisch mit Heptan/Essigester gereinigt. Es werden 1.6 g (28% Ausbeute) 4-Methoxy-2-methyl-5-(5-(trifluormethyl)pyrid-2-yl)pyrida-zin-3-on erhalten. ¹H-NMR (CDCl₃): 8.98 (s,1H), 8.39 (s,1H), 8.14 (d,1H), 8.02 (dq,1H), 4.28 (s,3H), 3.83 (s,3H).

Eine Mischung von 2.53 g (4.44 mmol) 4-Methoxy-2-methyl-5-(5-(trifluormethyl)pyrid-2-yl)pyridazin-3-on und 1.46 g (12.2 mmol) H₂O₂-Harnstoff in 20 ml Dichlorethan werden bei >5°C mit 1.72 ml (13.8 mmol) Trifluoressigsäureanhydrid versetzt und dann 2h bei Raumtemperatur gerührt. Anschließend wird mit Wasser und 1 M Natriumbisulfitlösung gewaschen, die organische Phase getrocknet und das Lösungsmittel im Vakuum entfernt. Der Rückstand 4-Methoxy-2-methyl-5-(5-(trifluormethyl)pyridin-1-oxid-2-yl)pyridazin-3-on wird ohne weitere Reinigung in die nächste Stufe eingesetzt (1.25 g, 94% Ausbeute).
¹H-NMR (CDCl₃): 8.58 (s,1H), 7.88 (s,1H), 7.55 (d,1H), 7.50 (d,1H), 4.24 (s,3H), 3.81 (s,3H).

4-Methoxy-2-methyl-5-(5-(trifluormethyl)pyridin-1-oxid-2-yl)pyridazin-3-on (4.15 mmol, roh) in 15 ml DMF werden mit 5.9 ml (42 mmol) Trifluoressigsäureanhydrid versetzt und 3h bei 90°C gerührt. Die Mischung wird dann auf Eiswasser gegeben und mit Essigester extrahiert. Die organische Phase wird mehrmals mit Wasser und ges. Kochsalzlösung gewaschen, getrocknet und das Lösungsmittel im Vakuum entfernt. Der Rückstand wird säulenchromatographisch mit Heptan/Essigester gereinigt. Es werden 0.41 g (33% Ausbeute) 4-Methoxy-2-methyl-5-(3-(trifluormethyl)pyrid-2-on-6-yl)pyridazin-3-on erhalten.
¹H-NMR (CDCl₃): 10.5 (brs,1H), 7.86 (d,1H), 7.84 (s,1H), 6.52 (d,1H), 4.42 (s,3H), 3.81 (s,3H).

0.38 g (1.28 mmol) 4-Methoxy-2-methyl-5-(3-(trifluormethyl)pyrid-2-on-6-yl)pyridazin-3-on und 0.22 g (1.6 mmol) Kaliumcarbonat in 5 ml DMF werden bei <5°C mit 0.19 g (1.5 mmol) Dimethylsulfat versetzt. Man lässt auf Raumtemperatur kommen und versetzt nach 6 h die Mischung mit Wasser und Essigester. Die organische Phase wird mehrmals mit 2N Salzsäure, dann mit ges. Natriumhydrogencarbonat-Lösung und mit Kochsalzlösung gewaschen. Nach Trocknen und Einengen wird der Rückstand säulenchromatographisch mit Heptan/Essigester gereinigt.
Fraktion A 0.26 g (65% Ausbeute) 4-Methoxy-2-methyl-5-(2-methoxy-3-(trifluormethyl)pyridin-6-yl)pyridazin-3-on
1H-NMR (CDCl₃): 8.42 (s,1H), 7.94 (d,1H), 7.75 (d,1H), 4.28 (s,3H), 4.09 (s,3H), 3.82 (s,3H).
Fraktion B 0.06 g (14% Ausbeute) 4-Methoxy-2-methyl-5-(1-methyl-3-(trifluormethyl)pyrid-2-on-6-yl)pyridazin-3-on
¹H-NMR (CDCl₃): 7.76 (d,1H), 7.55 (s,1H), 6.13 (d,1H), 4.29 (s,3H), 3.84 (s,3H), 3.43 (s,3H).

50 mg (0.16 mmol) 4-Methoxy-2-methyl-5-(1-methyl-3-(trifluormethyl)pyrid-2-on-6-yl)pyridazin-3-on in 1 ml Morpholin werden 1 h bei 100°C erhitzt. Anschließend wird mit Essigester aufgenommen und die Mischung mehrmals mit 2N Salzsäure und ges. Kochsalzlösung gewaschen. Nach Trocknen und Einengen erhält man die gewünschte Verbindung 4-Hydroxy-2-methyl-5-(1-methyl-3-(trifluormethyl)pyrid-2-on-6-yl)pyridazin-3-on. Ausbeute 50 mg (quant.).

Die in nachfolgender Tabelle aufgeführten Beispiele sind ganz besonders bevorzugt.

Die verwendeten Abkürzungen bedeuten:

| | | | | | | | |
|---|---|---|---|---|---|---|---|
| Me | = Methyl | Et | = Ethyl | Ph | = Phenyl | cPr | = cyclo-Propyl |

**Tabelle 1: Erfindungsgemäße Verbindungen der allgemeinen Formel (I), worin Q für Q¹ steht worin R² und R³ jeweils für Wassserstoff steht, und X¹ CZ¹, X² CW und X³ CH bedeuten.**

| | | | | | |
|---|---|---|---|---|---|
| 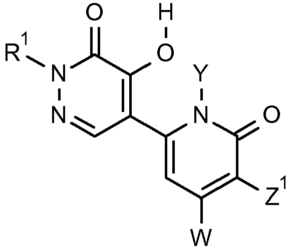 | | | | | |

| Nr. | R¹ | Y | Z¹ | W | Physikalische Daten (¹H-NMR, DMSO-d₆, 400 MHz) |
|---|---|---|---|---|---|
| 1-1 | Me | Me | H | H | |
| 1-2 | Me | Me | H | Me | |
| 1-3 | Me | Me | Me | H | |
| 1-4 | Me | Me | CF₃ | H | 7.97 (d,1H), 7.81 (s,1H), 6.38 (d,1H), 3.72 (s,3H), 3.33 (s,3H) |
| 1-5 | Me | Me | CF₃ | Me | |
| 1-6 | Me | Me | Ph | H | |
| 1-7 | Me | Me | 4-FPh | H | |
| 1-8 | Me | Me | 4-ClPh | H | |
| 1-9 | Me | Me | 3,5-F₂Ph | H | |
| 1-10 | Me | Me | Br | H | |
| 1-11 | Me | Me | Br | Me | |
| 1-12 | Me | Me | Cl | H | |
| 1-13 | Me | Me | Cl | Me | |
| 1-14 | Me | Et | CF₃ | H | |
| 1-15 | Me | CF₃CH₂ | CF₃ | H | |
| 1-16 | Me | MeOCH₂ | CF₃ | H | |
| 1-17 | Me | MeOCH₂CH₂ | CF₃ | H | |
| 1-18 | Me | allyl | CF₃ | H | |
| 1-19 | Me | propargyl | CF₃ | H | |
| 1-20 | Me | benzyl | CF₃ | H | |
| 1-21 | Me | Tetrahydrofuran -2-ylmethyl | CF₃ | H | |
| 1-22 | Me | Thiophen-2-ylmethyl | CF₃ | H | |
| 1-23 | Me | Et | Br | H | |
| 1-24 | Me | CF₃CH₂ | Br | H | |
| 1-25 | Me | MeOCH₂ | Br | H | |
| 1-26 | Me | MeOCH₂CH₂ | Br | H | |
| 1-27 | Me | allyl | Br | H | |
| 1-28 | Me | propargyl | Br | H | |
| 1-29 | Me | benzyl | Br | H | |
| 1-30 | Me | Tetrahydrofuran -2-ylmethyl | Br | H | |
| 1-31 | Me | Thiophen-2-ylmethyl | Br | H | |
| 1-32 | Et | Me | CF₃ | H | |
| 1-33 | allyl | Me | CF₃ | H | |
| 1-34 | propargyl | Me | CF₃ | H | |
| 1-35 | cPr | Me | CF₃ | H | |
| 1-36 | MeOCH₂ | Me | CF₃ | H | |
| 1-37 | Et | Et | CF₃ | H | |
| 1-38 | Et | Me | Br | H | |
| 1-39 | allyl | Me | Br | H | |
| 1-40 | propargyl | Me | Br | H | |
| 1-41 | cPr | Me | Br | H | |
| 1-42 | MeOCH₂ | Me | Br | H | |
| 1-43 | Et | Et | Br | H | |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigt beispielsweise die Verbindung Nr. 1-4 bei einer Aufwandmenge von 320 g/ha eine mindestens 80%-ige Wirkung gegen Cyperus esculentus, Abutilon theophrasti, Amaranthus retroflexus, Ipomoea purpurea, Stellaria media, Viola tricolor und Veronica persica.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigt beispielsweise die Verbindung Nr. 1-4 bei einer Aufwandmenge von 80 g/ha eine mindestens 80%-ige Wirkung gegen Abutilon theophrasti, Ipomoea purpurea, Stellaria media, Viola tricolor und Veronica persica.

## Patentansprüche

1. Pyridazinon-derivate der Formel (I) oder deren Salze 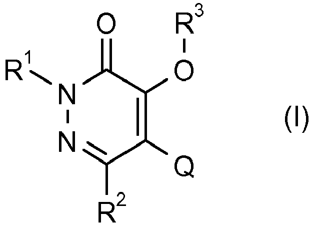 worin
Q bedeutet Q¹ oder Q², 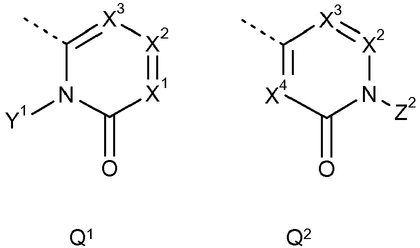
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, (C₃-C₆)-Cycloalkyl, (C₃-C₆)-Cycloalkyl-(C₁-C₆)-alkyl, (C₁-C₆)-Alkoxy-(C₁-C₆)-alkyl, Tetrahydropyran-2-yl oder durch s Reste R⁴ sustituiertes Benzyl,
R² bedeutet Wasserstoff, Halogen, Cyano, Hydroxy, Nitro, Amino,(C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₁-C₆)-alkyl-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, (C₁-C₆)-Alkoxy, Halogen-(C₁-C₆)-alkoxy, (C₁-C₆)-Alkyl-S(O)ₙ, Halogen-(C₁-C₆)-alkyl-S(O)ₙ, (C₁-C₆)-Alkyl-S(O)ₙ-(C₁-C₃)-alkyl, Halogen-(C₁-C₆)-alkyl-S(O)ₙ-(C₁-C₃)-alkyl, (C₁-C₆)-Alkylamino oder (C₁-C₆)-Dialkylamino,
R³ bedeutet Wasserstoff, (C₁-C₆)-Alkylcarbonyl, (C₁-C₆)-Alkoxycarbonyl, (C₁-C₆)-Alkyl-S(O)ₙ, (C₁-C₆)-Alkyl-S-carbonyl, Arycarbonyl oder Aryl S(O)ₙ bedeutet, wobei die zwei letztgenannten Reste jeweils durch s Reste R⁴ substituiert sind,
R⁴ bedeutet Halogen, (C₁-C₄)-Alkyl, Halogen-(C₁-C₄)-alkyl, (C₁-C₄)-Alkoxy,
X¹ bedeutet N oder CZ¹ ,
X² bedeutet N oder CW,
X³ bedeutet N oder CR⁵,
X⁴ bedeutet N oder CY²,
Y¹ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, OCOOR⁶, OC(O)N(R⁶)₂, OR⁶, OCOR⁶, OSO₂R⁷, (C₁-C₆)-Alkyl-S(O)ₙR⁷, (C₁-C₆)-Alkyl-OR⁶, (C₁-C₆)-Alkyl-OCOR⁶, (C₁-C₆)-Alkyl-OSO₂R⁷, (C₁-C₆)-Alkyl-CO₂R⁶, (C₁-C₆)-AlkylSO₂OR⁶, (C₁-C₆)-Alkyl-CON(R⁶)₂, (C₁-C₆)-Alkyl-CN, (C₁-C₆)-Alkyl-SO₂N(R⁶)₂, (C₁-C₆)-Alkyl-NR⁶COR⁶, (C₁-C₆)-Alkyl-NR⁶SO₂R⁷, CH₂P(O)(OR¹⁰)₂, (C₁-C₆)-Alkyl-Aryl, (C₁-C₆)-Alkyl-Heteroaryl, (C₁-C₆)-Alkyl-Heterocyclyl, wobei die drei letztgenannten Reste jeweils durch s Reste aus der Gruppe bestehend aus Halogen, Cyano, Nitro, (C₁-C₆)-Alkyl, Halogen-(C₁-C₆)-alkyl, S(O)ₙ-(C₁-C₆)-Alkyl, (C₁-C₆)-Alkoxy, Halogen-(C₁-C₆)-alkoxy substituiert sind, und wobei Heterocyclyl n Oxogruppen trägt,
Y² bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-AlkylS(O)ₙR², (C₁-C₆)-Alkyl-OR¹, (C₁-C₆)-Alkyl-OCOR¹, (C₁-C₆)-Alkyl-OSO₂R², (C₁-C₆)-Alkyl-CO₂R¹, (C₁-C₆)-Alkyl-CN, (C₁-C₆)-Alkyl-SO₂OR¹, (C₁-C₆)-Alkyl-CON(R¹)₂, (C₁-C₆)-Alkyl-SO₂N(R¹)₂, (C₁-C₆)-Alkyl-NR¹COR¹, (C₁-C₆)-Alkyl-NR¹SO₂R², N(R¹)₂, P(O)(OR⁵)₂, CH₂P(O)(OR⁵)₂, (C₁-C₆)-Alkyl-Phenyl, (C₁-C₆)-Alkyl-Heteroaryl, (C₁-C₆)-Alkyl-Heterocyclyl, Phenyl, Heteroaryl oder Heterocyclyl, wobei die sechs letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy, (C₁-C₆)-Alkoxy-(C₁-C₄)-alkyl und Cyanomethyl substituiert sind, und wobei Heterocyclyl n Oxogruppen trägt,
Z¹ bedeutet Wasserstoff, Halogen, Cyano, Rhodano, Nitro, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₁-C₆)-alkyl-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, P(O)(OR¹⁰)₂, Heteroaryl, Heterocyclyl oder Phenyl, wobei die drei letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl n Oxogruppen trägt,
Z² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₁-C₆)-alkyl-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, Heteroaryl, Heterocyclyl oder Phenyl, wobei die drei letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
W bedeutet Wasserstoff, Halogen, Cyano, Nitro, (C₁-C₄)-Alkyl, (C₁-C₄)-Haloalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, (C₁-C₄)-Alkoxy oder (C₁-C₄)-Haloalkoxy, oder
Z¹ oder Z² und W bilden gemeinsam mit den beiden Kohlenstoffatomen, an denen sie gebunden sind, einen fünf- oder sechsgliedrigen Ring bestehend aus t Kohlenstoffatomen und p Heteroatomen aus der Gruppe bestehend aus N, O und S, wobei dieser Ring durch q Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy, Halogen-(C₁-C₆)-alkoxy und (C₁-C₆)-Alkoxy-(C₁-C₆)-alkyl substituiert ist,
R⁵ bedeutet Wasserstoff, Halogen, (C₁-C₄)-Alkyl, Halogen-(C₁-C₄)-alkyl oder (C₁-C₄)-Alkoxy,
R⁶ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR⁸-Heteroaryl, (C₁-C₆)-Alkyl-NR⁸-Heterocyclyl wobei die 21 letztgenannten Reste durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO2R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
R⁷ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl, wobei diese 17 Reste durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
R⁸ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁹ bedeutet (C₁-C₆)-Alkyl, (C₂-C₆)-Alkenyl, (C₂-C₆)-Alkinyl oder Phenyl,
R¹⁰ bedeutet (C₁-C₄)-Alkyl,
n bedeutet 0, 1 oder 2,
p bedeutet 0, 1, 2 oder 3,
q bedeutet 0, 1, 2, 3 oder 4,
s bedeutet 0, 1, 2, 3, 4 oder 5,
t bedeutet 0, 1, 2, 3, 4, 5 oder 6.

2. Pyridazinon-derivate nach Anspruch 1, worin
Q bedeutet Q¹ oder Q²,
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, (C₃-C₆)-Cycloalkyl, (C₃-C₆)-Cycloalkyl-(C₁-C₆)-alkyl oder (C₁-C₆)-Alkoxy-(C₁-C₆)-alkyl,
R² bedeutet Wasserstoff, Halogen, Cyano, (C₁-C₆)-Alkyl, Halogen-(C₁-C₆)-alkyl, (C₂-C₆)-Alkenyl, (C₂-C₆)-Alkinyl oder(C₁-C₆)-Alkyl-S(O)ₙ,
R³ bedeutet Wasserstoff,
R⁴ bedeutet Halogen, (C₁-C₄)-Alkyl, Halogen-(C₁-C₄)-alkyl, (C₁-C₄)-Alkoxy,
X¹ bedeutet N oder CZ¹,
X² bedeutet N oder CW,
X³ bedeutet N oder CH,
X⁴ bedeutet N oder CY²,
Y¹ bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, OCOOR⁶, OC(O)N(R⁶)₂, OR⁶, OCOR⁶, OSO₂R⁷, (C₁-C₆)-Alkyl-S(O)ₙR⁷, (C₁-C₆)-Alkyl-OR⁶, (C₁-C₆)-Alkyl-OCOR⁶, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR⁶SO₂R⁷, CH₂P(O)(OR¹⁰)₂, (C₁-C₆)-Alkyl-Aryl, (C₁-C₆)-Alkyl-Heteroaryl, (C₁-C₆)-Alkyl-Heterocyclyl, wobei die drei letztgenannten Reste jeweils durch s Reste aus der Gruppe bestehend aus Halogen, Cyano, Nitro, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y² bedeutet Wasserstoff, Halogen, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, OCOOR⁶, OC(O)N(R⁶)₂, OR⁶, OCOR⁶, OSO₂R⁷, (C₁-C₆)-Alkyl-S(O)ₙR⁷, (C₁-C₆)-Alkyl-OR⁶, (C₁-C₆)-Alkyl-OCOR⁶, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR⁶SO₂R⁷, CH₂P(O)(OR¹⁰)₂, (C₁-C₆)-Alkyl-Phenyl, (C₁-C₆)-Alkyl-Heteroaryl, (C₁-C₆)-Alkyl-Heterocyclyl, wobei die drei letztgenannten Reste jeweils durch s Reste aus der Gruppe bestehend aus Halogen, Cyano, Nitro, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Z¹ bedeutet Wasserstoff, Halogen, Cyano, Rhodano, Nitro, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, P(O)(OR¹⁰)₂, Heteroaryl, Heterocyclyl oder Phenyl, wobei die letzten drei Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy oder Halogen-(C₁-C₆)-alkoxy substituiert sind, und wobei Heterocyclyl n Oxogruppen trägt,
Z² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, Heteroaryl, Heterocyclyl oder Phenyl, wobei die letzten drei Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
W bedeutet Wasserstoff, Halogen, (C₁-C₄)-Alkyl, (C₁-C₄)-Haloalkyl, (C₂-C₆)-Alkenyl, (C₂-C₆)-Alkinyl, (C₃-C₆)-Cycloalkyl, (C₁-C₆)-Alkyl-O-(C₁-C₆)-alkyl, (C₃-C₆)-Cycloalkyl-(C₁-C₆)-alkyl, (C₁-C₄)-Alkoxy oder (C₁-C₄)-Haloalkoxy,
R⁶ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR⁸-Heteroaryl, (C₁-C₆)-Alkyl-NR⁸-Heterocyclyl wobei die 21 letztgenannten Reste durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO2R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
R⁷ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl, wobei diese 17 Reste durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
R⁸ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁹ bedeutet (C₁-C₆)-Alkyl, (C₂-C₆)-Alkenyl, (C₂-C₆)-Alkinyl oder Phenyl,
R¹⁰ bedeutet (C₁-C₄)-Alkyl,
n bedeutet 0, 1 oder 2,
p bedeutet 0, 1, 2 oder 3,
q bedeutet 0, 1, 2, 3 oder 4,
s bedeutet 0, 1, 2, 3, 4 oder 5,
t bedeutet 0, 1, 2, 3, 4, 5 oder 6.

3. Pyridazinon-derivate nach Anspruch 1 oder 2, worin
Q bedeutet Q¹ oder Q²,
R¹ bedeutet (C₁-C₄)-Alkyl, Halogen-(C₁-C₃)-alkyl, (C₂-C₄)-Alkenyl, Propargyl, Cyclopropyl oder Cyclopropylmethyl,
R² bedeutet Wasserstoff, Halogen oder (C₁-C₆)-Alkyl,
R³ bedeutet Wasserstoff,
X¹ bedeutet N oder CZ¹,
X² bedeutet N oder CW,
X³ bedeutet N oder CH,
X⁴ bedeutet N oder CY²,
Y¹ bedeutet (C₁-C₆)-Alkyl, Halogen-(C₁-C₆)-alkyl, (C₃-C₆)-Cycloalkyl, OR⁶, S(O)ₙR⁷, (C₁-C₆)-Alkyl-S(O)ₙR⁷, (C₁-C₆)-Alkyl-OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR⁶SO₂R⁷, (C₁-C₆)-Alkyl-Heteroaryl, (C₁-C₆)-Alkyl-Heterocyclyl, wobei die beiden letztgenannten Reste jeweils durch s Reste aus der Gruppe bestehend aus Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Y² bedeutet (C₁-C₆)-Alkyl, Halogen-(C₁-C₆)-alkyl, (C₃-C₆)-Cycloalkyl, OR⁶, S(O)ₙR⁷, (C₁-C₆)-Alkyl-S(O)ₙR⁷, (C₁-C₆)-Alkyl-OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR⁶SO₂R⁷, (C₁-C₆)-Alkyl-Heteroaryl, (C₁-C₆)-Alkyl-Heterocyclyl, wobei die beiden letztgenannten Reste jeweils durch s Reste aus der Gruppe bestehend aus Halogen, (C₁-C₆)-Alkyl, Halogen-(C₁-C₆)-alkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Z¹ bedeutet Wasserstoff, Halogen, Cyano, Rhodano, Nitro, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, P(O)(OR¹⁰)₂, Heteroaryl, Heterocyclyl oder Phenyl, wobei die 3 letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
Z² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₂-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-Alkyl-S(O)ₙR⁷, (C₁-C₆)-Alkyl-OR⁶, (C₁-C₆)-Alkyl-OCOR¹, (C₁-C₆)-Alkyl-OSO₂R⁷, (C₁-C₆)-Alkyl-CO₂R⁶, (C₁-C₆)-Alkyl-SO₂OR⁶, (C₁-C₆)-Alkyl-CON(R⁶)₂, (C₁-C₆)-Alkyl-SO₂N(R⁶)₂, (C₁-C₆)-Alkyl-NR⁶COR⁶, (C₁-C₆)-Alkyl-NR¹SO₂R⁷, N(R⁶)₂, P(O)(OR¹⁰)₂, Heteroaryl, Heterocyclyl oder Phenyl, wobei die 3 letztgenannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, S(O)ₙ-(C₁-C₆)-Alkyl, (C₁-C₆)-Alkoxy und Halogen-(C₁-C₆)-alkoxy substituiert sind, und wobei Heterocyclyl n Oxogruppen trägt,
W bedeutet Wasserstoff, Halogen, (C₁-C₄)-Alkyl,
R⁶ bedeutet Wasserstoff, (C₁-C₆)-Alkyl, (C₁-C₆)-Halogenalkyl, (C₂-C₆)-Alkenyl, (C₂-C₆)-Halogenalkenyl, (C₂-C₆)-Alkinyl, (C₂-C₆)-Halogenalkinyl, (C₃-C₆)-Cycloalkyl, (C₃-C₆)-Cycloalkenyl, (C₃-C₆)-Halogencycloalkyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR⁸-Heteroaryl, (C₁-C₆)-Alkyl-NR⁸- Heterocyclyl wobei die 21 letztgenannten Reste jeweils durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO2R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
R⁷ bedeutet (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₁-C₆)-Alkyl-O-(C₁-C₆)-alkyl, (C₃-C₆)-Cycloalkyl-(C₁-C₆)-alkyl, Phenyl, Phenyl-(C₁-C₆)-alkyl, Heteroaryl, (C₁-C₆)-Alkyl-Heteroaryl, Heterocyclyl, (C₁-C₆)-Alkyl-Heterocyclyl, (C₁-C₆)-Alkyl-O-Heteroaryl, (C₁-C₆)-Alkyl-O-Heterocyclyl, (C₁-C₆)-Alkyl-NR³-Heteroaryl, (C₁-C₆)-Alkyl-NR³-Heterocyclyl, wobei diese 17 Reste jeweils durch s Reste aus der Gruppe bestehend aus Cyano, Halogen, Nitro, Rhodano, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ und (C₁-C₄)-Alkoxy-(C₂-C₆)-alkoxycarbonyl substituiert sind, und wobei Heterocyclyl n Oxogruppen trägt,
R⁸ bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁹ bedeutet (C₁-C₆)-Alkyl, (C₂-C₆)-Alkenyl, (C₂-C₆)-Alkinyl oder Phenyl,
R¹⁰ bedeutet (C₁-C₄)-Alkyl,
n bedeutet 0, 1 oder 2,
p bedeutet 0, 1, 2 oder 3,
q bedeutet 0, 1, 2, 3 oder 4,
s bedeutet 0, 1, 2, 3, 4 oder 5,
t bedeutet 0, 1, 2, 3, 4, 5 oder 6.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Herbizide Mittel gemäß Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

7. Herbizide Mittel gemäß Anspruch 6 enthaltend einen Safener.

8. Herbizide Mittel gemäß Anspruch 7 enthaltend cyprosulfamid, cloquintocetmexyl, mefenpyr-diethyl oder isoxadifen-ethyl.

9. Herbizide Mittel gemäß einem der Ansprüche 6 bis 8 enthaltend ein weiteres Herbizid.

10. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels gemäß einem der Ansprüche 4 bis 9 auf die Pflanzen oder auf den Ort des unerwünschten Pflanzenwachstums appliziert.

11. Verwendung von Verbindungen der Formel (I) gemäß einem der Ansprüche 1 bis 3 oder von herbiziden Mitteln gemäß einem der Ansprüche 4 bis 9 zur Bekämpfung unerwünschter Pflanzen.

12. Verwendung gemäß Anspruch 11, **dadurch gekennzeichnet, daß** die Verbindungen der Formel (I) zur Bekämpfung unerwünschter Pflanzen in Kulturen von Nutzpflanzen eingesetzt werden.

13. Verwendung gemäß Anspruch 12, **dadurch gekennzeichnet, daß** die Nutzpflanzen transgene Nutzpflanzen sind.

## Claims

1. Pyridazinone derivatives of the formula (I) or salts thereof 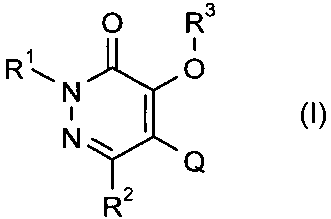 in which
Q is Q¹ or Q², 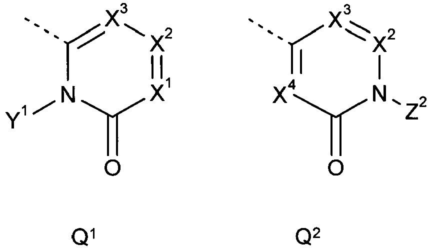
R¹ is hydrogen, (C₁-C₆)-alkyl, halo-(C₁-C₆)-alkyl, (C₂-C₆)-alkenyl, halo-(C₂-C₆)-alkenyl, (C₂-C₆)-alkynyl, (C₃-C₆)-cycloalkyl, (C₃-C₆)-cycloalkyl-(C₁-C₆)-alkyl, (C₁-C₆)-alkoxy-(C₁-C₆)-alkyl, tetrahydropyran-2-yl, or benzyl substituted by s R⁴ radicals,
R² is hydrogen, halogen, cyano, hydroxyl, nitro, amino, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₂-C₆)-alkynyl, (C₃-C₆)-cycloalkyl, halo-(C₃-C₆)-cycloalkyl, (C₁-C₆)-alkyl-(C₃-C₆)-cycloalkyl, (C₃-C₆)-cycloalkyl-(C₁-C₆)-alkyl, halo-(C₃-C₆)-cycloalkyl-(C₁-C₆)-alkyl, (C₁-C₆)-alkoxy, halo-(C₁-C₆)-alkoxy, (C₁-C₆)-alkyl-S(O)ₙ, halo-(C₁-C₆)-alkyl-S(O)ₙ, (C₁-C₆)-alkyl-S(O)ₙ-(C₁-C₃)-alkyl, halo-(C₁-C₆)-alkyl-S(O)ₙ-(C₁-C₃)-alkyl, (C₁-C₆)-alkylamino or (C₁-C₆)-dialkylamino,
R³ is hydrogen, (C₁-C₆)-alkylcarbonyl, (C₁-C₆)-alkoxycarbonyl, (C₁-C₆)-alkyl-S(O)ₙ, (C₁-C₆)-alkyl-S-carbonyl, arylcarbonyl or aryl S(O)ₙ, where the two latter radicals are each substituted by s R⁴ radicals,
R⁴ is halogen, (C₁-C₄)-alkyl, halo-(C₁-C₄)-alkyl, (C₁-C₄)-alkoxy,
X¹ is N or CZ¹,
X² is N or CW,
X³ is N or CR⁵,
X⁴ is N or CY²,
Y¹ is (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, OCOOR⁶, OC(O)N(R⁶)₂, OR⁶, OCOR⁶, OSO₂R⁷, (C₁-C₆-alkyl-S(O)ₙR⁷, (C₁-C₆)-alkyl-OR⁶, (C₁-C₆)-alkyl-OCOR⁶, (C₁-C₆)-alkyl-OSO₂R⁷, (C₁-C₆)-alkyl-CO₂R⁶, (C₁-C₆)-alkyl-SO₂OR⁶, (C₁-C₆)-alkyl-CON(R⁶)₂, (C₁-C₆)-alkyl-CN, (C₁-C₆)-alkyl-SO₂N(R⁶)₂, (C₁-C₆)-alkyl-NR⁶COR⁶, (C₁-C₆)-alkyl-NR⁶SO₂R⁷, CH₂P(O)(OR¹⁰)₂, (C₁-C₆)-alkylaryl, (C₁-C₆)-alkylheteroaryl, (C₁-C₆)-alkylheterocyclyl, where the three latter radicals are each substituted by s radicals from the group consisting of halogen, cyano, nitro, (C₁-C₆)-alkyl, halo-(C₁-C₆)-alkyl, S(O)ₙ-(C₁-C₆) - alkyl, (C₁-C₆) -alkoxy and halo-(C₁-C₆) -alkoxy, and where heterocyclyl bears n oxo groups,
Y² is hydrogen, nitro, halogen, cyano, thiocyanato, (C₁-C₆)-alkyl, halo-(C₁-C₆)-alkyl, (C₂-C₆)-alkenyl, halo-(C₂-C₆)-alkenyl, (C₂-C₆) -alkynyl, halo-(C₂-C₆)-alkynyl, (C₃-C₆)-cycloalkyl, (C₃-C₆)-cycloalkenyl, halo-(C₃-C₆) - cycloalkyl, (C₃-C₆)-cycloalkyl-(C₁-C₆)-alkyl, halo-(C₃-C₆)-cycloalkyl-(C₁-C₆)-alkyl, COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂ (C₁-C₆)-alkyl-S(O)ₙR², (C₁-C₆)-alkyl-OR¹, (C₁-C₆)-alkyl-OCOR¹, (C₁-C₆)-alkyl-OSO₂R², (C₁-C₆)-alkyl-CO₂R¹, (C₁-C₆)-alkyl-CN, (C₁-C₆)-alkyl-SO₂OR¹, (C₁-C₆)-alkyl-CON(R¹)₂, (C₁-C₆)-alkyl-SO₂N(R¹)₂, (C₁-C₆)-alkyl-NR¹COR¹, (C₁-C₆)-alkyl-NR¹SO₂R², N(R¹)₂, P(O)(OR⁵)₂, CH₂P(O)(OR⁵)₂, (C₁-C₆)-alkylphenyl, (C₁-C₆)-alkylheteroaryl, (C₁-C₆) - alkylheterocyclyl, phenyl, heteroaryl or heterocyclyl, where the six latter radicals are each substituted by s radicals from the group consisting of halogen, nitro, cyano, (C₁-C₆)-alkyl, halo-(C₁-C₆) -alkyl, (C₃-C₆)-cycloalkyl, S(O)ₙ-(C₁-C₆)-alkyl, (C₁-C₆)-alkoxy, halo-(C₁-C₆)-alkoxy, (C₁-C₆)-alkoxy-(C₁-C₄)-alkyl and cyanomethyl, and where heterocyclyl bears n oxo groups,
Z¹ is hydrogen, halogen, cyano, thiocyanato, nitro, (C₁-C₆)-alkyl, halo-(C₁-C₆)-alkyl, (C₂-C₆)-alkenyl, halo-(C₂-C₆)-alkenyl, (C₂-C₆) -alkynyl, halo-(C₂-C₆)-alkynyl, (C₃-C₆)-cycloalkyl, halo-(C₃-C₆)-cycloalkyl, (C₁-C₆)-alkyl-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR⁶, COOR⁶, OR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-alkyl-S(O)ₙR⁷, (C₁-C₆)-alkyl-OR⁶, (C₁-C₆)-alkyl-OCOR¹, (C₁-C₆)-alkyl-OSO₂R⁷, (C₁-C₆)-alkyl-CO₂R⁶, (C₁-C₆)-alkyl-SO₂OR⁶, (C₁-C₆)-alkyl-CON(R⁶)₂, (C₁-C₆)-alkyl-SO₂N(R⁶)₂, (C₁-C₆)-alkyl-NR⁶COR⁶, (C₁-C₆)-alkyl-NR¹SO₂R⁷, N(R⁶)₂, P(O)(OR¹⁰)₂, heteroaryl, heterocyclyl or phenyl, where the three latter radicals are each substituted by s radicals from the group consisting of halogen, nitro, cyano, (C₁-C₆)-alkyl, halo-(C₁-C₆-alkyl, (C₃-C₆)-cycloalkyl, S(O)ₙ-(C₁-C₆)-alkyl, (C₁-C₆)-alkoxy and halo-(C₁-C₆)-alkoxy, and where heterocyclyl bears n oxo groups,
Z² is (C₁-C₆)-alkyl, halo-(C₁-C₆)-alkyl, (C₂-C₆) - alkenyl, halo-(C₂-C₆)-alkenyl, (C₂-C₆)-alkynyl, halo-(C₂-C₆)-alkynyl, (C₃-C₆)-cycloalkyl, halo-(C₃-C₆)-cycloalkyl, (C₁-C₆)-alkyl-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR⁶, COOR⁶, OR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-alkyl-S(O)ₙR⁷, (C₁-C₆)-alkyl-OR⁶, (C₁-C₆)-alkyl-OCOR¹, (C₁-C₆)-alkyl-OSO₂R⁷, (C₁-C₆)-alkyl-CO₂R⁶, (C₁-C₆)-alkyl-SO₂OR⁶, (C₁-C₆)-alkyl-CON(R⁶)₂, (C₁-C₆)-alkyl-SO₂N(R⁶)₂, (C₁-C₆)-alkyl-NR⁶COR⁶, (C₁-C₆)-alkyl-NR¹SO₂R⁷, N(R⁶)₂, heteroaryl, heterocyclyl or phenyl, where the three latter radicals are each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy and halo-(C₁-C₆)-alkoxy, and where heterocyclyl bears n oxo groups,
W is hydrogen, halogen, cyano, nitro, (C₁-C₄)-alkyl, (C₁-C₄)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-halocycloalkyl, (C₁-C₆)-alkyl-O-(C₁-C₆)-alkyl, (C₃-C₆)-cycloalkyl-(C₁-C₆)-alkyl, (C₁-C₄)-alkoxy or (C₁-C₄)-haloalkoxy, or
Z¹ or Z² and W together with the two carbon atoms to which they are bonded form a five- or six-membered ring consisting of t carbon atoms and p heteroatoms from the group consisting of N, O and S, where this ring is substituted by q radicals from the group consisting of halogen, nitro, cyano, (C₁-C₆)-alkyl, halo-(C₁-C₆)-alkyl, (C₃-C₆) -cycloalkyl, S(O)ₙ-(C₁-C₆)-alkyl, (C₁-C₆)-alkoxy, halo-(C₁-C₆)-alkoxy and (C₁-C₆)-alkoxy-(C₁-C₆)-alkyl,
R⁵ is hydrogen, halogen, (C₁-C₄)-alkyl, halo-(C₁-C₄)-alkyl or (C₁-C₄)-alkoxy,
R⁶ is hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR⁸-heteroaryl, (C₁-C₆)-alkyl-NR⁸-heterocyclyl, where the 21 latter radicals are substituted by s radicals from the group consisting of cyano, halogen, nitro, thiocyanato, OR⁸, S(O)ₙR⁹, N(R⁸)₂, NR⁸OR⁸, COR⁸, OCOR⁸, SCOR⁹, NR⁸COR⁸, NR⁸SO2R⁹, CO₂R⁸, COSR⁹, CON(R⁸)₂ and (C₁-C₄-alkoxy-(C₂-C₆ - alkoxycarbonyl, and where heterocyclyl bears n oxo groups,
R⁷ is (C₁-C₆)-alkyl, (C₂-C₆)-alkenyl, (C₂-C₆)-alkynyl, (C₃-C₆)-cycloalkyl, (C₃-C₆)-cycloalkenyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocyclyl, (C₁-C₆)-alkyl-heterocyclyl, (C₁-C₆)-alkyl-O-heteroaryl, (C₁-C₆)-alkyl-O-heterocyclyl, (C₁-C₆)-alkyl-NR³-heteroaryl, (C₁-C₆)-alkyl-NR³-heterocyclyl, where these 17 radicals are substituted by s radicals from the group consisting of cyano, halogen, nitro, thiocyanato, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ and (C₁-C₄)-alkoxy-(C₂-C₆)-alkoxycarbonyl, and where heterocyclyl bears n oxo groups,
R⁸ is hydrogen, (C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl or phenyl,
R⁹ is (C₁-C₆)-alkyl, (C₂-C₆)-alkenyl, (C₂-C₆)-alkynyl or phenyl,
R¹⁰ is (C₁-C₄)-alkyl,
n is 0, 1 or 2,
p is 0, 1, 2 or 3,
q is 0, 1, 2, 3 or 4,
s is 0, 1, 2, 3, 4 or 5,
t is 0, 1, 2, 3, 4, 5 or 6.

2. Pyridazinone derivatives according to Claim 1, in which
Q is Q¹ or Q²,
R¹ is hydrogen, (C₁-C₆)-alkyl, halo-(C₁-C₆)-alkyl, (C₂-C₆)-alkenyl, halo-(C₂-C₆)-alkenyl, (C₂-C₆)-alkynyl, (C₃-C₆)-cycloalkyl, (C₃-C₆)-cycloalkyl-(C₁-C₆)-alkyl or (C₁-C₆)-alkoxy-(C₁-C₆)-alkyl,
R² is hydrogen, halogen, cyano, (C₁-C₆)-alkyl, halo-(C₁-C₆)-alkyl, (C₂-C₆)-alkenyl, (C₂-C₆)-alkynyl or (C₁-C₆)-alkyl-S(O)ₙ,
R³ is hydrogen,
R⁴ is halogen, (C₁-C₄)-alkyl, halo-(C₁-C₄)-alkyl, (C₁-C₄)-alkoxy,
X¹ is N or CZ¹,
X² is N or CW,
X³ is N or CH,
X⁴ is N or CY²,
Y¹ is (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, OCOOR⁶, OC(O)N(R⁶)₂, OR⁶, OCOR⁶, OSO₂R⁷, (C₁-C₆)-alkyl-S(O)ₙR⁷, (C₁-C₆)-alkyl-OR⁶, (C₁-C₆)-alkyl-OCOR⁶, (C₁-C₆) -alkyl-OSO₂R⁷, (C₁-C₆) -alkyl-CO₂R⁶, (C₁-C₆)-alkyl-SO₂OR⁶, (C₁-C₆)-alkyl-CON(R⁶)₂, (C₁-C₆)-alkyl-SO₂N(R⁶)₂, (C₁-C₆)-alkyl-NR⁶COR⁶, (C₁-C₆)-alkyl-NR⁶SO₂R⁷, CH₂P(O)(OR¹⁰)₂, (C₁-C₆)-alkylaryl, (C₁-C₆)-alkylheteroaryl, (C₁-C₆)-alkylheterocyclyl, where the three latter radicals are each substituted by s radicals from the group consisting of halogen, cyano, nitro, (C₁-C₆)-alkyl, halo-(C₁-C₆)-alkyl, S(O)ₙ-(C₁-C₆)-alkyl, (C₁-C₆)-alkoxy and halo-(C₁-C₆)-alkoxy, and where heterocyclyl bears n oxo groups,
Y² is hydrogen, halogen, (C₁-C₆-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, OCOOR⁶, OC(O)N(R⁶)₂, OR⁶, OCOR⁶, OSO₂R⁷, (C₁-C₆)-alkyl-S(O)ₙR⁷, (C₁-C₆)-alkyl-OR⁶, (C₁-C₆)-alkyl-OCOR⁶, (C₁-C₆)-alkyl-OSO₂R⁷, (C₁-C₆)-alkyl-CO₂R⁶, (C₁-C₆)-alkyl-SO₂OR⁶, (C₁-C₆)-alkyl-CON(R⁶)₂, (C₁-C₆)-alkyl-SO₂N(R⁶)₂, (C₁-C₆)-alkyl-NR⁶COR⁶, (C₁-C₆)-alkyl-NR⁶SO₂R⁷, CH₂P(O)(OR¹⁰)₂, (C₁-C₆)-alkylaryl, (C₁-C₆)-alkylheteroaryl, (C₁-C₆)-alkylheterocyclyl, where the three latter radicals are each substituted by s radicals from the group consisting of halogen, cyano, nitro, (C₁-C₆)-alkyl, halo-(C₁-C₆)-alkyl, S(O)ₙ-(C₁-C₆)-alkyl, (C₁-C₆)-alkoxy and halo-(C₁-C₆)-alkoxy, and where heterocyclyl bears n oxo groups,
Z¹ is hydrogen, halogen, cyano, thiocyanato, nitro, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₂-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆)-alkyl-S(O)ₙR⁷, (C₁-C₆)-alkyl-OR⁶, (C₁-C₆)-alkyl-OCOR¹, (C₁-C₆)-alkyl-OSO₂R⁷, (C₁-C₆)-alkyl-CO₂R⁶, (C₁-C₆)-alkyl-SO₂OR⁶, (C₁-C₆)-alkyl-CON(R⁶)₂, (C₁-C₆)-alkyl-SO₂N(R⁶)₂, (C₁-C₆)-alkyl-NR⁶COR⁶, (C₁-C₆)-alkyl-NR¹SO₂R⁷, N(R⁶)₂, P(O)(OR¹⁰)₂, heteroaryl, heterocyclyl or phenyl, where the latter three radicals are each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy and halo-(C₁-C₆)-alkoxy, and where heterocyclyl bears n oxo groups,
Z² is (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₂-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆) - cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₆) - alkyl-S(O)ₙR⁷, (C₁-C₆)-alkyl-OR⁶, (C₁-C₆)-alkyl-OCOR¹, (C₁-C₆)-alkyl-OSO₂R⁷, (C₁-C₆)-alkyl-CO₂R⁶, (C₁-C₆)-alkyl-SO₂OR⁶, (C₁-C₆)-alkyl-CON(R⁶)₂, (C₁-C₆)-alkyl-SO₂N(R⁶)₂, (C₁-C₆)-alkyl-NR⁶COR⁶, (C₁-C₆)-alkyl-NR¹SO₂R⁷, N(R⁶)₂, heteroaryl, heterocyclyl or phenyl, where the latter three radicals are each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy and halo-(C₁-C₆)-alkoxy, and where heterocyclyl bears n oxo groups,
W is hydrogen, halogen, (C₁-C₄)-alkyl, (C₁-C₄)-haloalkyl, (C₂-C₆)-alkenyl, (C₂-C₆)-alkynyl, (C₃-C₆)-cycloalkyl, (C₁-C₆)-alkyl-O-(C₁-C₆)-alkyl, (C₃-C₆)-cycloalkyl-(C₁-C₆)-alkyl, (C₁-C₄)-alkoxy or (C₁-C₄)-haloalkoxy,
R⁶ is hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-0-heterocyclyl, (C₁-C₆-alkyl-NR⁸-heteroaryl, (C₁-C₆)-alkyl-NR⁸-heterocyclyl, where the 21 latter radicals are substituted by s radicals from the group consisting of cyano, halogen, nitro, thiocyanato, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ and (C₁-C₄)-alkoxy-(C₂-C₆)-alkoxycarbonyl, and where heterocyclyl bears n oxo groups,
R⁷ is (C₁-C₆)-alkyl, (C₂-C₆)-alkenyl, (C₂-C₆)-alkynyl, (C₃-C₆)-cycloalkyl, (C₃-C₆)-cycloalkenyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocyclyl, (C₁-C₆)-alkyl-heterocyclyl, (C₁-C₆)-alkyl-O-heteroaryl, (C₁-C₆-alkyl-O-heterocyclyl, (C₁-C₆)-alkyl-NR³-heteroaryl, (C₁-C₆)-alkyl-NR³-heterocyclyl, where these 17 radicals are substituted by s radicals from the group consisting of cyano, halogen, nitro, thiocyanato, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ and (C₁-C₄)-alkoxy-(C₂-C₆)-alkoxycarbonyl, and where heterocyclyl bears n oxo groups,
R⁸ is hydrogen, (C₁-C₆)-alkyl, (C₂-C₆) -alkenyl, (C₂-C₆)-alkynyl, (C₃-C₆)-cycloalkyl, (C₃-C₆)-cycloalkyl-(C₁-C₆)-alkyl or phenyl,
R⁹ is (C₁-C₆)-alkyl, (C₂-C₆)-alkenyl, (C₂-C₆)-alkynyl or phenyl,
R¹⁰ is (C₁-C₄)-alkyl,
n is 0, 1 or 2,
p is 0, 1, 2 or 3,
q is 0, 1, 2, 3 or 4,
s is 0, 1, 2, 3, 4 or 5,
t is 0, 1, 2, 3, 4, 5 or 6.

3. Pyridazinone derivatives according to Claim 1 or 2, in which
Q is Q¹ or Q²,
R¹ is (C₁-C₄)-alkyl, halo-(C₁-C₃)-alkyl, (C₂-C₄)-alkenyl, propargyl, cyclopropyl or cyclopropylmethyl,
R² is hydrogen, halogen or (C₁-C₆)-alkyl,
R³ is hydrogen,
X¹ is N or CZ¹,
X² is N or CW,
X³ is N or CH,
X⁴ is N or CY²,
Y¹ is (C₁-C₆)-alkyl, halo-(C₁-C₆)-alkyl, (C₃-C₆)-cycloalkyl, OR⁶, S(O)ₙR⁷, (C₁-C₆)-alkyl-S(O)ₙR⁷, (C₁-C₆)-alkyl-OR⁶, (C₁-C₆)-alkyl-CON(R⁶)₂, (C₁-C₆)-alkyl-SO₂N(R⁶)₂, (C₁-C₆)-alkyl-NR⁶COR⁶, (C₁-C₆)-alkyl-NR⁶SO₂R⁷, (C₁-C₆)-alkylheteroaryl, (C₁-C₆)-alkylheterocyclyl, where the two latter radicals are each substituted by s radicals from the group consisting of halogen, (C₁-C₆)-alkyl, halo-(C₁-C₆)-alkyl, S(O)ₙ-(C₁-C₆)-alkyl, (C₁-C₆)-alkoxy and halo-(C₁-C₆)-alkoxy, and where heterocyclyl bears n oxo groups,
Y² is (C₁-C₆-alkyl, halo-(C₁-C₆)-alkyl, (C₃-C₆)-cycloalkyl, OR⁶, S(O)ₙR⁷, (C₁-C₆)-alkyl-S(O)ₙR⁷, (C₁-C₆)-alkyl-OR⁶, (C₁-C₆)-alkyl-CON(R⁶)₂, (C₁-C₆)-alkyl-SO₂N(R⁶)₂, (C₁-C₆)-alkyl-NR⁶COR⁶, (C₁-C₆)-alkyl-NR⁶SO₂R⁷, (C₁-C₆)-alkylheteroaryl, (C₁-C₆)-alkylheterocyclyl, where the two latter radicals are each substituted by s radicals from the group consisting of halogen, (C₁-C₆)-alkyl, halo-(C₁-C₆)-alkyl, S(O)ₙ-(C₁-C₆)-alkyl, (C₁-C₆)-alkoxy and halo-(C₁-C₆)-alkoxy, and where heterocyclyl bears n oxo groups,
Z¹ is hydrogen, halogen, cyano, thiocyanato, nitro, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₂-C₆)-alkynyl, (C₃-C₆)-cycloalkyl, halo-(C₃-C₆)-cycloalkyl, (C₃**-**C₆)-cycloalkyl-(C₁-C₆)-alkyl, halo-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, SOₙR⁷, SO₂OR⁶, SO₂N(R⁶)₂, NR⁶SO₂R⁷, NR⁶COR⁶, (C₁-C₆)-alkyl-S(O)ₙR⁷, (C₁-C₆)-alkyl-OR⁶, (C₁-C₆)-alkyl-OCOR¹, (C₁-C₆)-alkyl-OSO₂R⁷, (C₁-C₆)-alkyl-CO₂R⁶, (C₁-C₆)-alkyl-SO₂OR⁶, (C₁-C₆)-alkyl-CON(R⁶)₂, (C₁-C₆)-alkyl-SO₂N(R⁶)₂, (C₁-C₆)-alkyl-NR⁶COR⁶, (C₁-C₆)-alkyl-NR¹SO₂R⁷, N(R⁶)₂, P(O)(OR¹⁰)₂, heteroaryl, heterocyclyl or phenyl, where the 3 latter radicals are each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy and halo- (C₁-C₆) -alkoxy, and where heterocyclyl bears n oxo groups,
Z² is (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₂-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, (C₁-C₅)-alkyl-S(O)ₙR⁷, (C₁-C₆) -alkyl-OR⁶, (C₁-C₆)-alkyl-OCOR¹, (C₁-C₆)-alkyl-OSO₂R⁷, (C₁-C₆)-alkyl-CO₂R⁶, (C₁-C₆)-alkyl-SO₂OR⁶, (C₁-C₆)-alkyl-CON(R⁶)₂, (C₁-C₆)-alkyl-SO₂N(R⁶)₂, (C₁-C₆)-alkyl-NR⁶COR⁶, (C₁-C₆)-alkyl-NR¹SO₂R⁷, N(R⁶)₂, P(O)(OR¹⁰)₂, heteroaryl, heterocyclyl or phenyl, where the 3 latter radicals are each substituted by s radicals from the group consisting of halogen, nitro, cyano, (C₁-C₆)-alkyl, halo-(C₁-C₆)-alkyl, (C₃-C₆)-cycloalkyl, S(O)ₙ-(C₁-C₆)-alkyl, (C₁-C₆)-alkoxy and halo-(C₁-C₆)-alkoxy, and where heterocyclyl bears n oxo groups,
W is hydrogen, halogen, (C₁-C₄)-alkyl,
R⁶ is hydrogen, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-cycloalkenyl, (C₃-C₆)-halocycloalkyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆-alkyl-NR⁸-heteroaryl, (C₁-C₆)-alkyl-NR⁸-heterocyclyl, where the 21 latter radicals are each substituted by s radicals from the group consisting of cyano, halogen, nitro, thiocyanato, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ and (C₁-C₄)-alkoxy-(C₂-C₆)-alkoxycarbonyl, and where heterocyclyl bears n oxo groups,
R⁷ is (C₁-C₆)-alkyl, (C₂-C₆)-alkenyl, (C₂-C₆)-alkynyl, (C₃-C₆)-cycloalkyl, (C₃-C₆)-cycloalkenyl, (C₁-C₆)-alkyl-O-(C₁-C₆)-alkyl, (C₃-C₆)-cycloalkyl-(C₁-C₆)-alkyl, phenyl, phenyl-(C₁-C₆)-alkyl, heteroaryl, (C₁-C₆)-alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR³-heteroaryl, (C₁-C₆)-alkyl-NR³-heterocyclyl, where these 17 radicals are each substituted by s radicals from the group consisting of cyano, halogen, nitro, thiocyanato, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ and (C₁-C₄)-alkoxy-(C₂-C₆)-alkoxycarbonyl, and where heterocyclyl bears n oxo groups,
R⁸ is hydrogen, (C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl or phenyl,
R⁹ is (C₁-C₆)-alkyl, (C₂-C₆)-alkenyl, (C₂-C₆)-alkynyl or phenyl,
R¹⁰ is (C₁-C₄)-alkyl,
n is 0, 1 or 2,
p is 0, 1, 2 or 3,
q is 0, 1, 2, 3 or 4,
s is 0, 1, 2, 3, 4 or 5,
t is 0, 1, 2, 3, 4, 5 or 6.

4. Herbicidal composition **characterized by** a herbicidally active content of at least one compound of the formula (I) according to any of Claims 1 to 3.

5. Herbicidal composition according to Claim 4 in a mixture with formulation auxiliaries.

6. Herbicidal composition according to Claim 4 or 5, comprising at least one further pesticidally active substance from the group consisting of insecticides, acaricides, herbicides, fungicides, safeners, and growth regulators.

7. Herbicidal composition according to Claim 6, comprising a safener.

8. Herbicidal composition according to Claim 7, comprising cyprosulfamide, cloquintocet-mexyl, mefenpyr-diethyl or isoxadifen-ethyl.

9. Herbicidal composition according to any of Claims 6 to 8, comprising a further herbicide.

10. Method for controlling unwanted plants, **characterized in that** an effective amount of at least one compound of the formula (I) according to any of Claims 1 to 3 or of a herbicidal composition according to any of Claims 4 to 9 is applied to the plants or to the site of the unwanted vegetation.

11. Use of compounds of the formula (I) according to any of Claims 1 to 3 or of herbicidal compositions according to any of Claims 4 to 9 for controlling unwanted plants.

12. Use according to Claim 11, **characterized in that** the compounds of the formula (I) are used for controlling unwanted plants in crops of useful plants.

13. Use according to Claim 12, **characterized in that** the useful plants are transgenic useful plants.

## Revendications

1. Dérivés de pyridazinone de formule (I) ou leurs sels 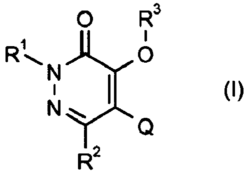 dans lesquels
Q signifie Q¹ ou Q² 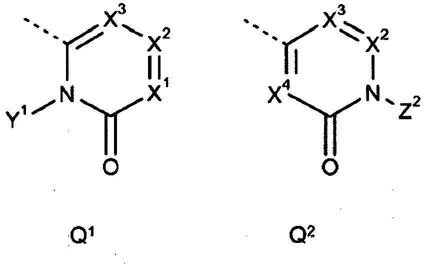
R¹ signifie hydrogène, alkyle en (C₁-C₆), halogéno-alkyle en (C₁-C₆), alcényle en (C₂-C₆), halogéno-alcényle en (C₂-C₆), alcynyle en (C₂-C₆), cycloalkyle en (C₃-C₆), cycloalkyle en (C₃-C₆)-alkyle en (C₁-C₆), alcoxy en (C₁-C₆)-alkyle en (C₁-C₆), tétrahydropyran-2-yle ou benzyle substitué par s radicaux R⁴,
R² signifie hydrogène, halogène, cyano, hydroxy, nitro, amino, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), halogéno-cycloalkyle en (C₃-C₆), alkyle en (C₁-C₆)-cycloalkyle en (C₃-C₆), cycloalkyle en (C₃-C₆)-alkyle en (C₁-C₆), halogéno-cycloalkyle en (C₃-C₆)-alkyle en (C₁-C₆), alcoxy en (C₁-C₆), halogéno-alcoxy en (C₁-C₆), alkyle en (C₁-C₆)-S(O)ₙ, halogéno-alkyle en (C₁-C₆)-S(O)ₙ, alkyle en (C₁-C₆)-S(O)ₙ-alkyle en (C₁-C₃), halogéno-alkyle en (C₁-C₆)-S(O)ₙ-alkyle en (C₁-C₃), alkylamino en (C₁-C₆) ou dialkylamino en (C₁-C₆),
R³ signifie hydrogène, alkylcarbonyle en (C₁-C₆), alcoxycarbonyle en (C₁-C₆), alkyle en (C₁-C₆)-S(O)ₙ, alkyle en (C₁-C₆)-S-carbonyle, arylcarbonyle ou aryleS(O)ₙ, les deux derniers radicaux cités étant chacun substitués par s radicaux R⁴,
R⁴ signifie halogène, alkyle en (C₁-C₄), halogéno-alkyle en (C₁-C₄), alcoxy en C₁-C₄),
X¹ signifie N ou CZ¹,
X² signifie N ou CW,
X³ signifie N ou CR⁵,
X⁴ signifie N ou CY²,
Y¹ signifie alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), OCOOR⁶, OC(O)N(R⁶)₂, OR⁶, OCOR⁶, OSO₂R⁷, alkyle en (C₁-C₆)-S(O)ₙR⁷, alkyle en (C₁-C₆)-OR⁶, alkyle en (C₁-C₆)-OCOR⁶, alkyle en (C₁-C₆)-OSO₂R⁷, alkyle en (C₁-C₆)-CO₂R⁶, alkyle en (C₁-C₆)-SO₂OR⁶, alkyle en (C₁-C₆)-CON(R⁶)₂, alkyle en (C₁-C₆)-CN, alkyle en (C₁-C₆)-SO₂N(R⁶)₂, alkyle en (C₁-C₆)-NR⁶COR⁶, alkyle en (C₁-C₆)-NR⁶SO₂R⁷, CH₂P(O)(OR¹⁰)₂, alkyle en (C₁-C₆)-aryle, alkyle en (C₁-C₆)-hétéroaryle, alkyle en (C₁-C₆)-hétérocyclyle, les trois derniers radicaux cités étant chacun substitués par s radicaux du groupe constitué par halogène, cyano, nitro, alkyle en (C₁-C₆), halogéno-alkyle en (C₁-C₆), S(O)ₙ-alkyle en (C₁-C₆), alcoxy en (C₁-C₆), halogéno-alcoxy en (C₁-C₆), et l'hétérocyclyle portant n groupes oxo,
Y² signifie hydrogène, nitro, halogène, cyano, rhodano, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), cycloalcényle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), COR¹, COOR¹, OCOOR¹, NR¹COOR¹, C(O)N(R¹)₂, NR¹C(O)N(R¹)₂, OC(O)N(R¹)₂, CO(NOR¹)R¹, NR¹SO₂R², NR¹COR¹, OR¹, OSO₂R², S(O)ₙR², SO₂OR¹, SO₂N(R¹)₂-alkyle en (C₁-C₆)-S(O)ₙR², alkyle en (C₁-C₆)-OR¹, alkyle en (C₁-C₆)-OCOR¹, alkyle en (C₁-C₆)-OSO₂R², alkyle en (C₁-C₆)-CO₂R¹, alkyle en (C₁-C₆)-CN, alkyle en (C₁-C₆)-SO₂OR¹, alkyle en (C₁-C₆)-CON(R¹)₂, alkyle en (C₁-C₆)-SO₂N(R¹)₂, alkyle en (C₁-C₆)-NR¹COR¹, alkyle en (C₁-C₆)-NR¹SO₂R², N(R¹)₂, P(O)(OR⁵)₂, CH₂P(O)(OR⁵)₂, alkyle en (C₁-C₆)-phényle, alkyle en (C₁-C₆)-hétéroaryle, alkyle en (C₁-C₆)-hétérocyclyle, phényle, hétéroaryle ou hétérocyclyle, les six derniers radicaux cités étant chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆), alcoxy en (C₁-C₆-alkyle en (C₁-C₄) et cyanométhyle, et l'hétérocyclyle portant n groupes oxo,
Z¹ signifie hydrogène, halogène, cyano, rhodano, nitro, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), halogéno-cycloalkyle en (C₃-C₆), alkyle en (C₁-C₆)-cycloalkyle en (C₃-C₆), cycloalkyle en (C₃-C₆)-alkyle en (C₁-C₆), halogéno-cycloalkyle en (C₃-C₆)-alkyle en (C₁-C₆), COR⁶, COOR⁶, OR⁶, OCOOR⁶, NR⁶COOR⁶, C(O)N(R⁶)₂, NR⁶C(O)N(R6)², OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, alkyle en (C₁-C₆)-S(O)ₙR⁷, alkyle en (C₁-C₆)-OR⁶, alkyle en (C₁-C₆)-OCOR¹, alkyle en (C₁-C₆)-OSO₂R⁷, alkyle en (C₁-C₆)-COaR⁶, alkyle en (C₁-C₆)-SO₂OR⁶, alkyle en (C₁-C₆)-CON(R⁶)₂, alkyle en (C₁-C₆)-SO₂N(R⁶)₂, alkyle en (C₁-C₆)-NR⁶COR⁶, alkyle en (C₁-C₆)-NR¹SO₂R⁷, N(R⁶)₂, P(O)(OR¹⁰)₂, hétéroaryle, hétérocyclyle ou phényle, les trois derniers radicaux cités étant chacun substitué par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆) ou halogéno-alcoxy en (C₁-C₆), et l'hétérocyclyle portant n groupes oxo,
Z² signifie alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), halogéno-cycloalkyle en (C₃-C₆), alkyle en (C₁-C₆)-cycloalkyle en (C₃-C₆), cycloalkyle en (C₃-C₆) -alkyle en (C₁-C₆)halogéno-cycloalkyle en (C₃-C₆)-alkyle en (C₁-C₆), COR⁶, COOR⁶, OR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, alkyle en (C₁-C₆)-S(O)ₙR⁷, alkyle en (C₁-C₆)-OR⁶, alkyle en (C₁-C₆)-OCOR¹, alkyle en (C₁-C₆)-OSO₂R⁷, alkyle en (C₁-C₆)-CO₂R⁶, alkyle en (C₁-C₆)-SO₂OR⁶, alkyle en (C₁-C₆)-CON(R⁶)₂, alkyle en (C₁-C₆)-SO₂N(R⁶)₂, alkyle en (C₁-C₆)-NR⁶COR⁶, alkyle en (C₁-C₆)-NR¹SO₂R⁷, N(R⁶)₂, hétéroaryle, hétérocyclyle ou phényle, les trois derniers radicaux cités étant chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆) et halogéno-alcoxy en (C₁-C₆), et l'hétérocyclyle portant n groupes oxo,
W signifie hydrogène, halogène, cyano, nitro, alkyle en (C₁-C₄), haloalkyle en (C₁-C₄), alcényle en (C₂-C₆), halogénoalcényle en (C₂-C₆), alcynyle en (C₂-C₆), halogénoalcynyle en (C₂-C₆), cycloalkyle en (C₃-C₆),
halogénocycloalkyle en (C₃-C₆), alkyle en (C₁-C₆)-O-alkyle en (C₁-C₆), cycloalkyle en (C₃-C₆)-alkyle en (C₁-C₆), alcoxy en (C₁-C₄) ou haloalcoxy en (C₁-C₄), ou
Z¹ ou Z² et W forment ensemble avec les deux atomes de carbone auxquels ils sont reliés un cycle à cinq ou six éléments constitué par t atomes de carbone et p hétéroatomes du groupe constitué par N, O et S, ce cycle étant substitué par q radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆), halogéno-alcoxy en (C₁-C₆) et alcoxy en (C₁-C₆)-alkyle en (C₁-C₆), R⁵ signifie hydrogène, halogène, alkyle en (C₁-C₄), halogéno-alkyle en (C₁-C₄) ou alcoxy en (C₁-C₄),
R⁶ signifie hydrogène, alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₂-C₆), cycloalkyle en (C₃-C₆), cycloalcényle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR⁸-hétéroaryle, alkyle en (C₁-C₆)-NR⁸-hétérocyclyle, les 21 derniers radicaux cités étant substitués par s radicaux du groupe constitué par cyano, halogène, nitro, rhodano, OR⁸, S(O)ₙR⁹, N(R⁸)₂, NR⁸OR⁸, COR⁸, COR⁸, SCOR⁹, NR⁸COR⁸, NR⁸SO₂R⁹, CO₂R⁸, COSR⁹, CON(R⁸)₂ et alcoxy en (C₁-C₄)-alcoxycarbonyle en (C₂-C₆), et l'hétérocyclyle portant n groupes oxo,
R⁷ signifie alkyle en (C₁-C₆), alcényle en (C₂-C₆), alcynyle en (C₂-C₆), cycloalkyle en (C₃-C₆), cycloalcényle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆-O-hétérocyclyle, alkyle en (C₁-C₆)-NR³-hétéroaryle, alkyle en (C₁-C₆)-NR³-hétérocyclyle, ces 17 radicaux étant substitués par s radicaux du groupe constitué par cyano, halogène, nitro, rhodano, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ et alcoxy en (C₁-C₄)-alcoxycarbonyle en (C₂-C₆), et l'hétérocyclyle portant n groupes oxo,
R⁸ signifie hydrogène, alkyle en (C₁-C₆), alcényle en (C₂-C₆), alcynyle en (C₂-C₆), cycloalkyle en (C₃-C₆), cycloalkyle en (C₃-C₆)-alkyle en (C₁-C₆) ou phényle,
R⁹ signifie alkyle en (C₁-C₆), alcényle en (C₂-C₆), alcynyle en (C₂-C₆) ou phényle,
R¹⁰ signifie alkyle en (C₁-C₄),
n signifie 0, 1 ou 2,
p signifie 0, 1, 2 ou 3,
q signifie 0, 1, 2, 3 ou 4,
s signifie 0, 1, 2, 3, 4 ou 5,
t signifie 0, 1, 2, 3, 4, 5 ou 6.

2. Dérivés de pyridazinone selon la revendication 1, dans lesquels
Q signifie Q¹ ou Q²,
R¹ signifie hydrogène, alkyle en (C₁-C₆), halogéno-alkyle en (C₁-C₆), alcényle en (C₂-C₆), halogéno-alcényle en (C₂-C₆), alcynyle en (C₂-C₆), cycloalkyle en (C₃-C₆), cycloalkyle en (C₃-C₆)-alkyle en (C₁-C₆) ou alcoxy en (C₁-C₆)-alkyle en (C₁-C₆),
R² signifie hydrogène, halogène, cyano, alkyle en (C₁-C₆), halogéno-alkyle en (C₁-C₆), alcényle en (C₂-C₆), alcynyle en (C₂-C₆) ou alkyle en (C₁-C₆)-S(O)ₙ,
R³ signifie hydrogène,
R⁴ signifie halogène, alkyle en (C₁-C₄), halogéno-alkyle en (C₁-C₄), alcoxy en (C₁-C₄),
X¹ signifie N ou CZ¹,
X² signifie N ou CW,
X³ signifie N ou CH,
X⁴ signifie N ou CY²,
Y¹ signifie alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), OCOOR⁶, OC(O)N(R⁶)₂, OR⁶, OCOR⁶, OSO₂R⁷, alkyle en (C₁-C₆)-S(O)ₙR⁷, alkyle en (C₁-C₆)-OR⁶, alkyle en (C₁-C₆)-OCOR⁶, alkyle en (C₁-C₆)-OSO₂R⁷, alkyle en (C₁-C₆)-CO₂R⁶, alkyle en (C₁-C₆)-SO₂OR⁶, alkyle en (C₁-C₆)-CON(R⁶)₂, alkyle en (C₁-C₆)-SO₂N(R⁶)₂, alkyle en (C₁-C₆)-NR⁶COR⁶, alkyle en (C₁-C₆)-NR⁶SO₂R⁷, CH₂P(O)(OR¹⁰)₂, alkyle en (C₁-C₆)-aryle, alkyle en (C₁-C₆)-hétéroaryle, alkyle en (C₁-C₆)-hétérocyclyle, les trois derniers radicaux cités étant chacun substitués par s radicaux du groupe constitué par halogène, cyano, nitro, alkyle en (C₁-C₆), halogéno-alkyle en (C₁-C₆), S(O)ₙ-alkyle en (C₁-C₆), alcoxy en (C₁-C₆), halogéno-alcoxy en (C₁-C₆), et l'hétérocyclyle portant n groupes oxo,
Y² signifie hydrogène, halogène, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), OCOOR⁶, OC(O)N(R⁶)₂, OR⁶, OCOR⁶, OSO₂R⁷, alkyle en (C₁-C₆)-S(O)ₙR⁷, alkyle en (C₁-C₆)-OR⁶, alkyle en (C₁-C₆)-OCOR⁶, alkyle en (C₁-C₆)-OSO₂R⁷, alkyle en (C₁-C₆)-CO₂R⁶, alkyle en (C₁-C₆)-SO₂OR⁶, alkyle en (C₁-C₆)-CON(R⁶)₂, alkyle en (C₁-C₆)-SO₂N(R⁶)₂, alkyle en (C₁-C₆)-NR⁶COR⁶, alkyle en (C₁-C₆)-NR⁶SO₂R⁷, CH₂P(O)(OR¹⁰)₂, alkyle en (C₁-C₆)-phényle, alkyle en (C₁-C₆)-hétéroaryle, alkyle en (C₁-C₆)-hétérocyclyle, les trois derniers radicaux cités étant chacun substitués par s radicaux du groupe constitué par halogène, cyano, nitro, alkyle en (C₁-C₆), halogéno-alkyle en (C₁-C₆), S(O)ₙ-alkyle en (C₁-C₆), alcoxy en (C₁-C₆) et halogéno-alcoxy en (C₁-C₆), et l'hétérocyclyle portant n groupes oxo,
Z¹ signifie hydrogène, halogène, cyano, rhodano, nitro, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆-alkyle en (C₁-C₆), COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, alkyle en (C₁-C₆)-S(O)ₙR⁷, alkyle en (C₁-C₆)-OR⁶, alkyle en (C₁-C₆)-OCOR¹, alkyle en (C₁-C₆)-OSO₂R⁷, alkyle en (C₁-C₆)-CO₂R⁶, alkyle en (C₁-C₆)-S₂OR⁶, alkyle en (C₁-C₆)-CON(R⁶)₂, alkyle en (C₁-C₆)-SO₂N(R⁶)₂, alkyle en (C₁-C₆) -NR⁶COR⁶, alkyle en (C₁-C₆)-NR¹SO₂R⁷, N(R⁶)₂, P(O)(OR¹⁰)₂, hétéroaryle, hétérocyclyle ou phényle, les trois derniers radicaux cités étant chacun substitué par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆) ou halogéno-alcoxy en (C₁-C₆), et l'hétérocyclyle portant n groupes oxo,
Z² signifie alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, alkyle en (C₁-C₆)-S(O)ₙR⁷, alkyle en (C₁-C₆)-OR⁶, alkyle en (C₁-C₆)-OCOR¹, alkyle en (C₁-C₆)-OSO₂R⁷, alkyle en (C₁-C₆)-CO₂R⁶, alkyle en (C₁-C₆)-SO₂OR⁶, alkyle en (C₁-C₆)-CON(R⁶)₂, alkyle en (C₁-C₆)-SO₂N(R⁶)₂, alkyle en (C₁-C₆)-NR⁶COR⁶, alkyle en (C₁-C₆)-NR¹SO₂R⁷, N(R⁶)₂, hétéroaryle, hétérocyclyle ou phényle, les trois derniers radicaux étant chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆) et halogéno-alcoxy en (C₁-C₆), et l'hétérocyclyle portant n groupes oxo,
W signifie hydrogène, halogène, alkyle en (C₁-C₄), haloalkyle en (C₁-C₄), alcényle en (C₂-C₆), alcynyle en (C₂-C₆), cycloalkyle en (C₃-C₆), alkyle en (C₁-C₆)-O-alkyle en (C₁-C₆), cycloalkyle en (C₃-C₆)-alkyle en (C₁-C₆), alcoxy en (C₁-C₄) ou haloalcoxy en (C₁-C₄),
R⁶ signifie hydrogène, alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₂-C₆), cycloalkyle en (C₃-C₆), cycloalcényle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR⁸-hétéroaryle, alkyle en (C₁-C₆)-NR⁸-hétérocyclyle, les 21 derniers radicaux cités étant substitués par s radicaux du groupe constitué par cyano, halogène, nitro, rhodano, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)2 et alcoxy en (C₁-C₄)-alcoxycarbonyle en (C₂-C₆), et l'hétérocyclyle portant n groupes oxo,
R⁷ signifie alkyle en (C₁-C₆), alcényle en (C₂-C₆), alcynyle en (C₂-C₆), cycloalkyle en (C₃-C₆), cycloalcényle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR³-hétéroaryle, alkyle en (C₁-C₆)-NR³-hétérocyclyle, ces 17 radicaux étant substitués par s radicaux du groupe constitué par cyano, halogène, nitro, rhodano, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ et alcoxy en (C₁-C₄)-alcoxycarbonyle en (C₂-C₆), et l'hétérocyclyle portant n groupes oxo,
R⁸ signifie hydrogène, alkyle en (C₁-C₆), alcényle en (C₂-C₆), alcynyle en (C₂-C₆), cycloalkyle en (C₃-C₆), cycloalkyle en (C₃-C₆)-alkyle en (C₁-C₆) ou phényle,
R⁹ signifie alkyle en (C₁-C₆), alcényle en (C₂-C₆), alcynyle en (C₂-C₆) ou phényle,
R¹⁰ signifie alkyle en (C₁-C₄),
n signifie 0, 1 ou 2,
p signifie 0, 1, 2 ou 3,
q signifie 0, 1, 2, 3 ou 4,
s signifie 0, 1, 2, 3, 4 ou 5,
t signifie 0, 1, 2, 3, 4, 5 ou 6.

3. Dérivés de pyridazinone selon la revendication 1 ou 2, dans lesquels
Q signifie Q¹ ou Q²,
R¹ signifie alkyle en (C₁-C₄), halogéno-alkyle en (C₁-C₃), alcényle en (C₂-C₄), propargyle, cycloprople ou cyclopropylméthyle,
R² signifie hydrogène, halogène ou alkyle en (C₁-C₆),
R³ signifie hydrogène,
X¹ signifie N ou CZ¹,
X² signifie N ou CW,
X³ signifie N ou CH,
X⁴ signifie N ou CY²,
Y¹ signifie alkyle en (C₁-C₆), halogéno-alkyle en (C₁-C₆), cycloalkyle en (C₃-C₆), OR⁶, S(O)ₙR⁷, alkyle en (C₁-C₆)-S(O)ₙR⁷, alkyle en (C₁-C₆)-OR⁶, alkyle en (C₁-C₆)-CON(R⁶)₂, alkyle en (C₁-C₆)-SO₂N(R⁶)₂, alkyle en (C₁-C₆)-NR⁶COR⁶, alkyle en (C₁-C₆)-NR⁶SO₂R⁷, alkyle en (C₁-C₆)-hétéroaryle, alkyle en (C₁-C₆)-hétérocyclyle, les deux derniers radicaux cités étant chacun substitués par s radicaux du groupe constitué par halogène, alkyle en (C₁-C₆), halogéno-alkyle en (C₁-C₆), S(O)ₙ-alkyle en (C₁-C₆), alcoxy en (C₁-C₆) et halogéno-alcoxy en (C₁-C₆), et l'hétérocyclyle portant n groupes oxo,
Y² signifie alkyle en (C₁-C₆), halogéno-alkyle en (C₁-C₆), cycloalkyle en (C₃-C₆), OR⁶, S(O)ₙR⁷, alkyle en (C₁-C₆)-S(O)ₙR⁷, alkyle en (C₁-C₆)-OR⁶, alkyle en (C₁-C₆)-CON(R⁶)₂, alkyle en (C₁-C₆)-SO₂N(R⁶)₂, alkyle en (C₁-C₆)-NR⁶COR⁶, alkyle en (C₁-C₆)-NR⁶SO₂R⁷, alkyle en (C₁-C₆)-hétéroaryle, alkyle en (C₁-C₆)-hétérocyclyle, les deux derniers radicaux cités étant chacun substitués par s radicaux du groupe constitué par halogène, alkyle en (C₁-C₆), halogéno-alkyle en (C₁-C₆), S(O)ₙ-alkyle en (C₁-C₆), alcoxy en (C₁-C₆) et halogéno-alcoxy en (C₁-C₆), et l'hétérocyclyle portant n groupes oxo,
Z¹ signifie hydrogène, halogène, cyano, rhodano, nitro, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, alkyle en (C₁-C₆)-S(O)ₙR⁷, alkyle en (C₁-C₆)-OR⁶, alkyle en (C₁-C₆)-OCOR¹, alkyle en (C₁-C₆)-OSO₂R⁷, alkyle en (C₁-C₆)-CO₂R⁶, alkyle en (C₁-C₆)-SO₂OR⁶, alkyle en (C₁-C₆)-CON(R⁶)₂, alkyle en (C₁-C₆)-SO₂N(R⁶)₂, alkyle en (C₁-C₆)-NR⁶COR⁶, alkyle en (C₁-C₆)-NR¹SO₂R⁷, N(R⁶)₂, P(O)(OR¹⁰)₂, hétéroaryle, hétérocyclyle ou phényle, les 3 derniers radicaux cités étant chacun substitué par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆) et halogéno-alcoxy en (C₁-C₆), et l'hétérocyclyle portant n groupes oxo,
Z² signifie alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₂-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆-alkyle en (C₁-C₆), COR⁶, COOR⁶, OCOOR⁶, NR⁶COOR⁶, C(O)N(R⁶)₂, NR⁶C(O)N(R⁶)₂, OC(O)N(R⁶)₂, C(O)NR⁶OR⁶, OSO₂R⁷, S(O)ₙR⁷, SO₂OR⁶, SO₂N(R⁶)₂, NR⁶SO₂R⁷, NR⁶COR⁶, alkyle en (C₁-C₆)-S(O)ₙR⁷, alkyle en (C₁-C₆)-OR⁶, alkyle en (C₁-C₆)-OCOR¹, alkyle en (C₁-C₆)-OSO₂R⁷, alkyle en (C₁-C₆)-CO₂R⁶, alkyle en (C₁-C₆)-SO₂OR⁶, alkyle en (C₁-C₆)-CON(R⁶)₂, alkyle en (C₁-C₆)-SO₂N(R⁶)₂, alkyle en (C₁-C₆-NR⁶COR⁶, alkyle en (C₁-C₆) -NR¹SO₂R⁷, N(R⁶)₂, P(O)(OR¹⁰)₂, hétéroaryle, hétérocyclyle ou phényle, les 3 derniers radicaux cités étant chacun substitués par s radicaux du groupe constitué par halogène, nitro, cyano, alkyle en (C₁-C₆), halogéno-alkyle en (C₁-C₆), cycloalkyle en (C₃-C₆), S(O)ₙ-alkyle en (C₁-C₆), alcoxy en (C₁-C₆) et halogéno-alcoxy en (C₁-C₆), et l'hétérocyclyle portant n groupes oxo,
W signifie hydrogène, halogène, alkyle en (C₁-C₄).
R⁶ signifie hydrogène, alkyle en (C₁-C₆), halogénoalkyle en (C₁-C₆), alcényle en (C₂-C₆), halogénoalcényle en (C₂-C₆), alcynyle en (C₂-C₆), halogénoalcynyle en (C₂-C₆), cycloalkyle en (C₃-C₆), cycloalcényle en (C₃-C₆), halogénocycloalkyle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆) -hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR⁸-hétéroaryle, alkyle en (C₁-C₆)-NR⁸-hétérocyclyle, les 21 derniers radicaux cités étant chacun substitués par s radicaux du groupe constitué par cyano, halogène, nitro, rhodano, OR⁸, S(O)ₙR⁹, N(R⁸)₂, NR⁸OR⁸, COR⁸, OCOR⁸, SCOR⁹, NR⁸COR⁸, NR⁸SO₂R⁹, CO₂R⁸, COSR⁹, CON(R⁸)₂ et alcoxy en (C₁-C₄)-alcoxycarbonyle en (C₂-C₆), et l'hétérocyclyle portant n groupes oxo,
R⁷ signifie alkyle en (C₁-C₆), alcényle en (C₂-C₆, alcynyle en (C₂-C₆), cycloalkyle en (C₃-C₆),
cycloalcényle en (C₃-C₆), alkyle en (C₁-C₆)-O-alkyle en (C₁-C₆), cycloalkyle en (C₃-C₆)-alkyle en (C₁-C₆), phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR³-hétéroaryle, alkyle en (C₁-C₆)-NR³-hétérocyclyle, ces 17 radicaux étant chacun substitués par s radicaux du groupe constitué par cyano, halogène, nitro, rhodano, OR⁸, S(O)ₙR⁹, N(R⁸)₂, NR⁸OR⁸, COR⁸, COR⁸, SCOR⁹, NR⁸COR⁸, NR⁸SO₂R⁹, CO₂R⁸, COSR⁹, CON(R⁸)₂ et alcoxy en (C₁-C₄)-alcoxycarbonyle en (C₂-C₆), et l'hétérocyclyle portant n groupes oxo,
R⁸ signifie hydrogène, alkyle en (C₁-C₆), alcényle en (C₂-C₆), alcynyle en (C₂-C₆), cycloalkyle en (C₃-C₆), cycloalkyle en (C₃-C₆)-alkyle en (C₁-C₆) ou phényle,
R⁹ signifie alkyle en (C₁-C₆), alcényle en (C₂-C₆), alcynyle en (C₂-C₆) ou phényle,
R¹⁰ signifie alkyle en (C₁-C₄),
n signifie 0, 1 ou 2,
p signifie 0, 1, 2 ou 3,
q signifie 0, 1, 2, 3 ou 4,
s signifie 0, 1, 2, 3, 4 ou 5,
t signifie 0, 1, 2, 3, 4, 5 ou 6.

4. Agent herbicide, **caractérisé par** une teneur herbicide efficace en au moins un composé de formule (I) selon l'une quelconque des revendications 1 à 3.

5. Agent herbicide selon la revendication 4, en mélange avec des adjuvants de formulation.

6. Agent herbicide selon la revendication 4 ou 5, contenant au moins un agent actif pesticide supplémentaire du groupe constitué par les insecticides, les acaricides, les herbicides, les fongicides, les agents protecteurs et les régulateurs de croissance.

7. Agent herbicide selon la revendication 6, contenant un agent protecteur.

8. Agent herbicide selon la revendication 7, contenant du cyprosulfamide, du cloquintocet-mexyl, du méfenpyr-diéthyle ou de l'isoxadifène-éthyle.

9. Agent herbicide selon l'une quelconque des revendications 6 à 8, contenant un herbicide supplémentaire.

10. Procédé de lutte contre des plantes indésirables, **caractérisé en ce qu'**une quantité efficace d'au moins un composé de formule (I) selon l'une quelconque des revendications 1 à 3 ou d'un agent herbicide selon l'une quelconque des revendications 4 à 9 est appliquée sur les plantes ou à l'emplacement de la végétation indésirable.

11. Utilisation de composés de formule (I) selon l'une quelconque des revendications 1 à 3 ou d'agents herbicides selon l'une quelconque des revendications 4 à 9 pour lutter contre des plantes indésirables.

12. Utilisation selon la revendication 11, **caractérisée en ce que** les composés de formule (I) sont utilisés pour lutter contre des plantes indésirables dans des cultures de plantes utiles.

13. Utilisation selon la revendication 12, **caractérisée en ce que** les plantes utiles sont des plantes utiles transgéniques.
